# EUROPEAN PATENT SPECIFICATION

(11) **EP 2 471 785 B1**
(45) Date of publication and mention of the grant of the patent: **19.04.2017**
(21) Application number: 12161959.7
(22) Date of filing: 15.10.2008
(51) Int. Cl.: C07D 333/26, C09B 49/00, C09B 49/06

(54) **Novel thiophene-based dye and preparation thereof**
Neuer Farbstoff auf Thiophenbasis und Herstellung davon
Nouveau colorant à base de Thiophène et sa préparation

(30) Priority: 15.10.2007 KR 20070103629; 26.12.2007 KR 20070137908; 25.04.2008 KR 20080038731; 30.06.2008 KR 20080062482
(43) Date of publication of application: 04.07.2012
(62) Divisional of application: 08839120.6
(73) Proprietor: Dongjin Semichem Co., Ltd., Seo-gu Incheon 404-205 (KR)
(72) Inventor: Bae, Ho-Gi, 451-781 Pyeongtaek-city Gyeonggi-do (KR); Lee, Chong-chan, 363-883 Chungcheongbuk-do (KR); Kim, Jong-bok, 138-172 Seoul (KR); Moon, Hyoung-don, 447-270 Osan City Gyeonggi-do (KR); Park, Tae-jin, 405-759 Incheon (KR); Baek, Jong-hyub, 139-224 Seoul (KR); Yang, Hoe-taek, 445-923 Hwaseong-city Gyeonggi-do (KR); An, Hyun-cheol, 445-931 Hwaseong-city Gyeonggi-do (KR)
(74) Representative: Bockhorni & Kollegen

(56) References cited:
- WO-A1-2007/100033
- RUIKUI CHEN ET AL: "Effect of Tetrahydroquinoline Dyes Structure on the Performance of Organic Dye-Sensitized Solar Cells", CHEMISTRY OF MATERIALS, vol. 19, no. 16, 1 August 2007 (2007-08-01), pages 4007-4015, XP55027153, ISSN: 0897-4756, DOI: 10.1021/cm070617g

## Description

### [Technical Field]

The present invention relates to a dithienothiophene-based dye, a dye-sensitized photoelectric conversion element and a dye-sensitized solar cell.

### [Background Art]

Since a research team of Michael Grätzel at the Ecole Polytechnique Federale de Lausanne (EPFL) in Switzerland developed a dye-sensitive nano particle titanium dioxide solar cell in 1991, lots of studies have been conducted on the area. The dye-sensitized solar cell provides higher efficiency and requires significantly lower manufacturing costs than an existing silicon solar cell does, and can possibly replace an existing amorphous silicon solar cell. Unlike a silicon solar cell, the dye-sensitized solar cell is a photoelectrochemical solar cell which includes a dye molecule absorbing visible rays to generate an electron-hole pair and a transition metal oxide transferring a generated electron, as main materials.

Ruthenium metal complexes which are widely used in the dye-sensitized solar cell cost too much while providing high photoelectric conversion efficiency. Recently, it has been found that a metal-free organic dye, which has good properties from the perspective of the light absorption efficiency, stable oxidation-reduction reaction and charge-transfer (CT) absorption within molecules, can be used in the solar cell instead of expensive ruthenium metal complexes. Thus, there have been full-scale studies for the metal-free organic dye.

The organic dye generally includes a structure of electron donor-electron acceptor end groups which are connected by a π-coupling unit. In most of organic dyes, an amine derivative acts as an electron donor and 2-cyanoacrylic acid or rhodanine end groups act as an electron acceptor, both of which are connected by π-coupling system such as metaphosphorus unit or thiophene chain.

Typically, a structural change in the amine unit as the electron donor brings about electronic property change, e.g. a light absorption spectrum shifted to blue color, and changes the length of the π-conjugation to adjust the light absorption spectrum and redox potential.

However, as most of organic dyes known provide lower conversion efficiency and driving stability than the ruthenium metal complexes do, there has been continuous efforts to develop a new dye that provides better molar extinction coefficient and higher photoelectric conversion efficiency than existing organic dye compounds do, by changing the type or the length of π-conjugation of the electron donor and electron acceptor.

Further state of the art is known, namely RUIKUI CHEN ET AL, "Effect of Tetrahydroquinoline Dyes Structure on the Performance of Organic Dye-Sensitized Solar Cells", CHEMISTRY OF MATERIALS, (20070801), vol. 19, no. 16, doi:10.1021/cm070617g, ISSN 0897-4756, pages 4007 - 4015, XP055027153 and WO 2007/100033 A1 in which dithienophene dyes for use in solar cells and other applications are disclosed.

### [Disclosure]

### [Technical Problem]

Accordingly, it is an aspect of the present invention to provide an organic dye which has a better molar extinction coefficient and higher photoelectric conversion efficiency than a conventional metal complex dye does to significantly enhance efficiency of a solar cell, and a preparation thereof.

Also, it is another aspect of the present invention to provide a dye-sensitized photoelectric conversion element which provides enhanced photoelectric conversion efficiency and good J_{sc} (short circuit photocurrent density) and molar extinction coefficient by including the dye, and a solar cell having better efficiency.

Additional aspects and/or advantages of the present invention will be set forth in part in the description which follows and, in part, will be obvious from the description, or may be learned by practice of the present invention.

### [Technical Solution]

The foregoing and/or other aspects of the present invention are also achieved by providing a dithienothiophene-based dye which is represented by a chemical formula 7 below which comprises one of the compounds 158 to 180 discussed further below 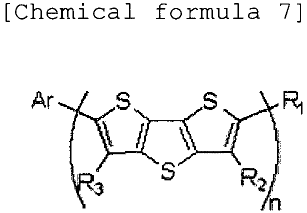
wherein, Ar is alkyl, aryl, alkoxy or heteroaryl of C₁₋₅₀ which is substituted or not substituted by alkyl, alkoxy, halogen, amide, cyano, hydroxyl, nitro, acyl, aryl or heteroaryl group of C₁₋₅₀;
R₁ is 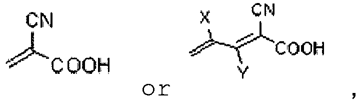 and X and Y comprise hydrogen or C₁₋₃₀ alkyl, C₁₋₃₀ alkoxy or C₁₋₃₀ heteroalkoxy, independently;
R₂ and R₃ comprise hydrogen, halogen, amide, cyano, hydroxyl, nitro, acyl, C₁₋₃₀ alkyl, C₁₋₃₀ alkoxy, C₁₋₃₀ alkylcarbonyl or C₆₋₂₀ aryl, independently, and these are coupled with each other to form an oxygen-containing hetero ring if they are C₁₋₃₀ alkoxy; and
n is an integer number from 1 to 10.

The foregoing and/or other aspects of the present invention are also achieved by providing a dye-sensitized photoelectric conversion element which comprises oxide semiconductor particles applied with the compound represented by the chemical formula 7 which includes a structure of compounds 158 to 180 below.

The foregoing and/or other aspects of the present invention are also achieved by providing a dye-sensitized solar cell which comprises the dye-sensitized photoelectric conversion element.

### [Advantageous Effects]

A thiophene-based dye according to the present invention may greatly enhance efficiency of a solar cell by providing better molar extinction coefficient, J_{sc} (short-circuit photocurrent density) and photoelectric conversion efficiency than a conventional metal complex dye and drastically lower dye synthesis cost by being purified without expensive column.

### [Mode for Invention]

Hereinafter, exemplary embodiments of the present invention will be described with reference to accompanying drawings, wherein like numerals refer to like elements and repetitive descriptions will be avoided as necessary.

Hereinafter, the present invention will be described in detail.

The present inventors have discovered that a dye sensitized solar cell which is manufactured by applying a thiophene dye compound which is represented by chemical formulas 7 including a structure of compounds 158 to 180 having a new organic dye structure, to oxide semiconductor particles, provides better efficiency with higher photoelectric conversion efficiency, Jsc (short circuit photocurrent density) and molar extinction coefficient than an existing dye-sensitized solar cell does, and have completed the present invention.

Further, the present invention provides a dithienothiophene-based dye which is represented by a chemical formula 7 below.

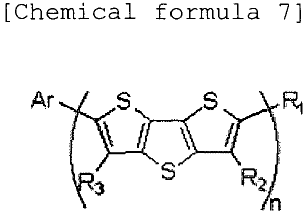

In the chemical formula 7, Ar is alkyl, aryl, alkoxy or heteroaryl of C₁₋₅₀ which is substituted or not substituted by alkyl, alkoxy, halogen, amide, cyano, hydroxyl, nitro, acyl, aryl or heteroaryl group of C₁₋₅₀;
R₁ is 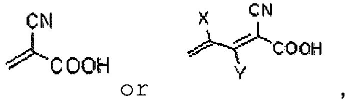 and X and Y is hydrogen or C₁₋₃₀ alkyl, C₁₋₃₀ alkoxy or C₁₋₃₀ heteroalkoxy, independently;
R₂ and R₃ are halogen, amide, cyano, hydroxyl, nitro, acyl, C₁₋₃₀ alkyl, C₁₋₃₀ alkoxy, C₁₋₃₀ alkylcarbonyl or C₆₋₂₀ aryl, independently, and these are coupled with each other to form an oxygen-containing hetero ring if they are C₁₋₃₀ alkoxy; and
n is a positive number from 1 to 10.

Preferably, Ar is 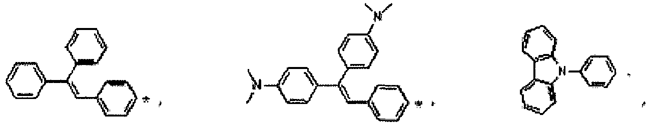 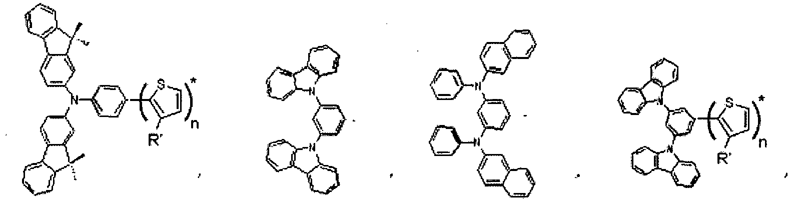 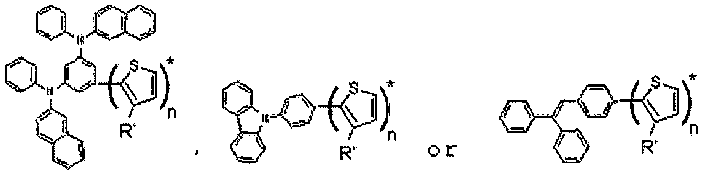 (here, R' is alkyl having 1 to 6 carbon atoms, n is a positive number from 0 to 5 and * is a coupling, independently), rings of aryl and heteroaryl groups may have at least one substituent therein, and the substituent may include alkyl group, alkoxy group, halogen atom, amide group, cyano group, hydroxyl group, nitro group, acyl group, aryl group or heterogryl of C₁₋₃₀, independently.

The organic dye which is represented by the chemical formula 7 provides a dithienothiophene-based dye which includes a structure of compounds 158 to 180.

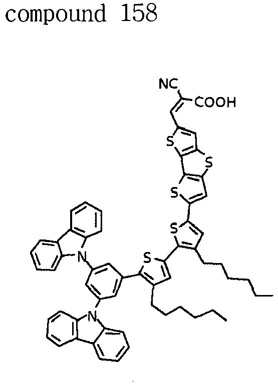

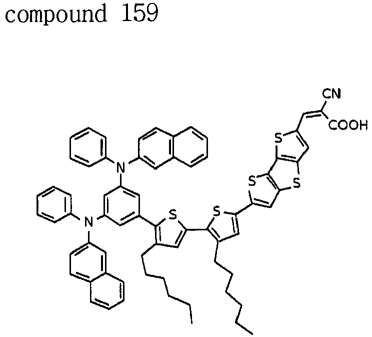

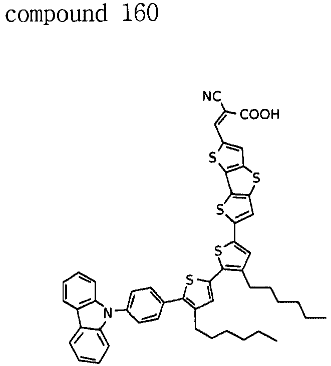

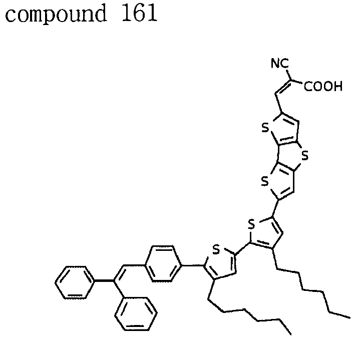

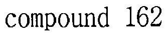

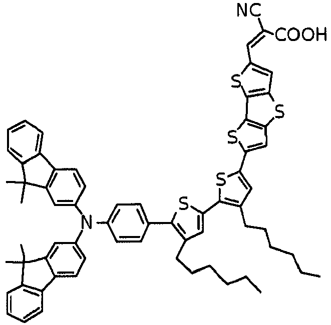

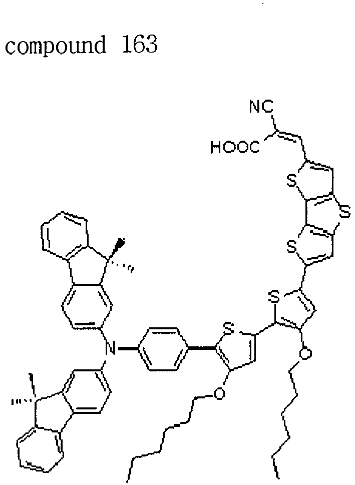

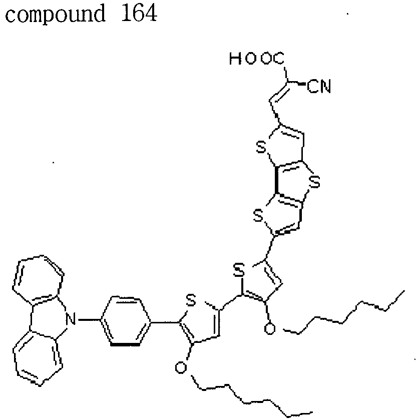

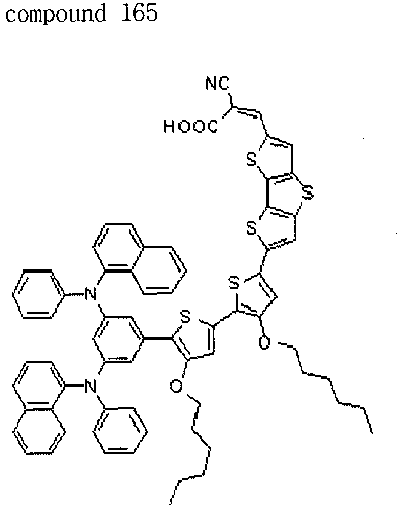

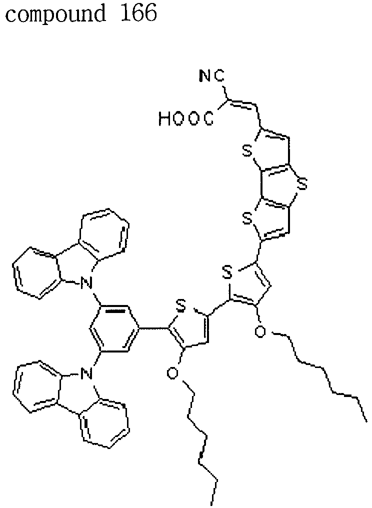

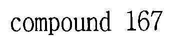

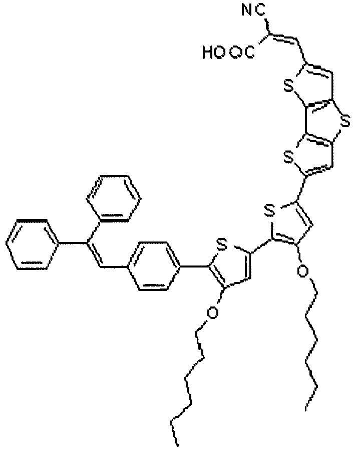

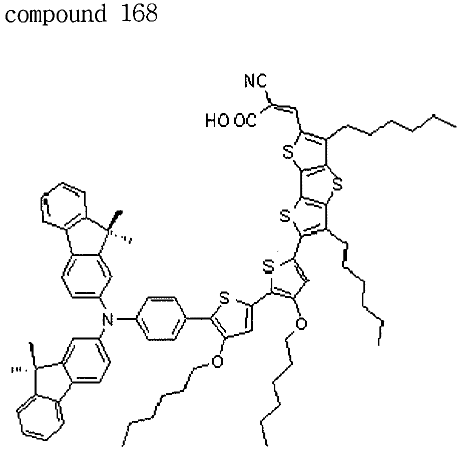

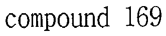

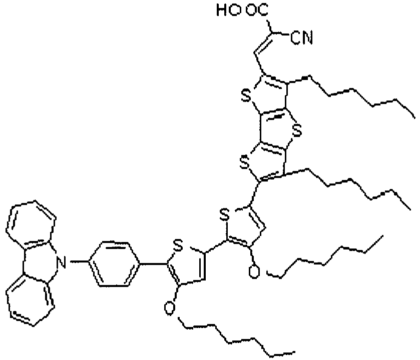

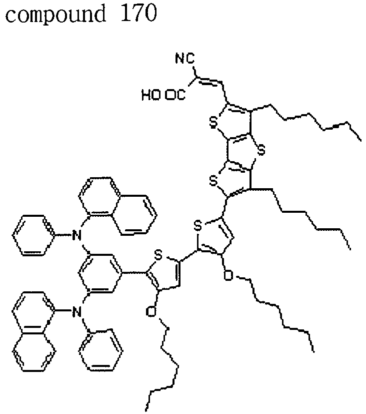

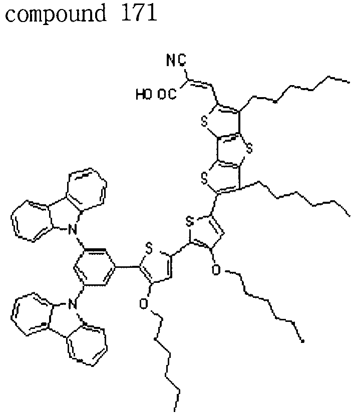

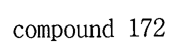

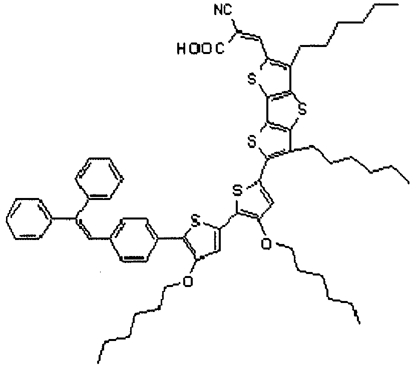

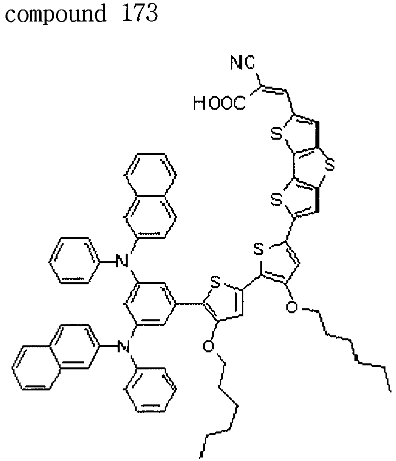

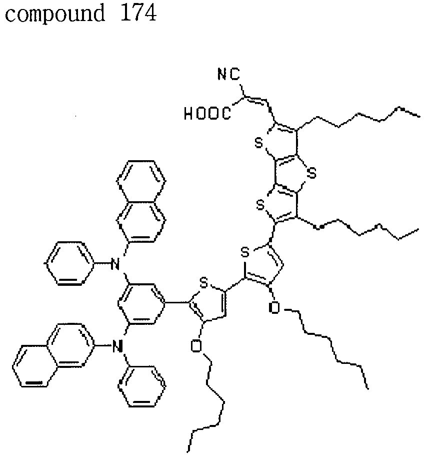

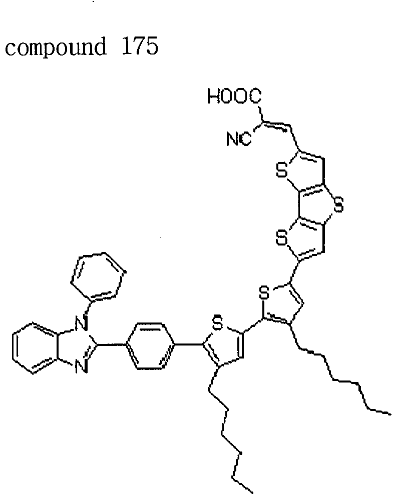

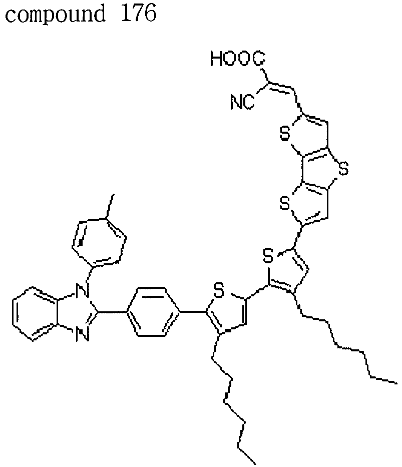

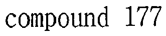

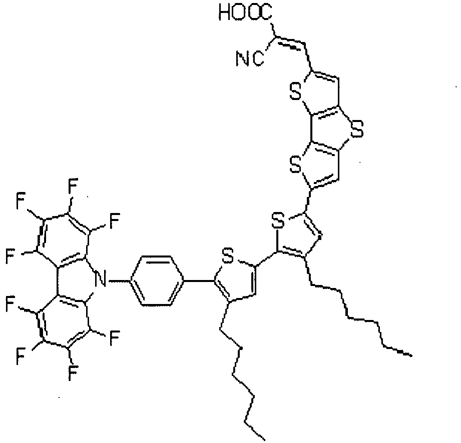

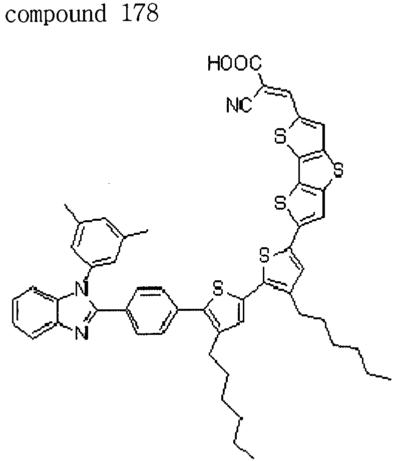

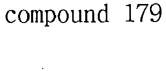

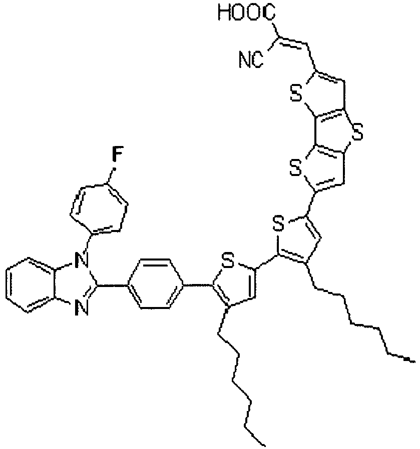

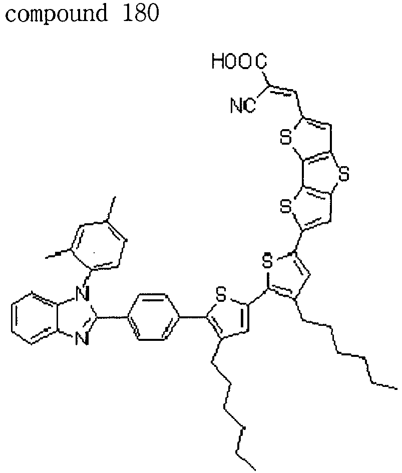

Further, the present invention provides a preparation of the dye which is represented by the chemical formula 7. The dye which is represented by the chemical formula 7 may be prepared by 1) having a compound in a chemical formula 8 below Suzuki-coupling-react to a compound in a chemical formula 9 below to make a compound in a chemical formula 10, 2) having the compound in the chemical formula 10 react to POC1₃ of dimethylformamide to make a compound in a chemical formula 11 below and 3) having the compound in the chemical formula 11 react to cyanoacetic acid or to a compound in a chemical formula 12 below under a presence of piperidine of CH₃CN. A detailed example of the preparation may be represented by a reaction formula 1 below.

### [Chemical formula 8]

### Ar-Br

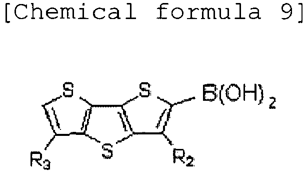

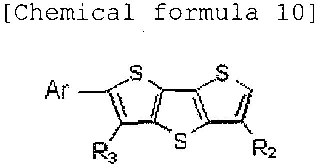

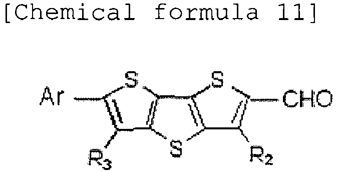

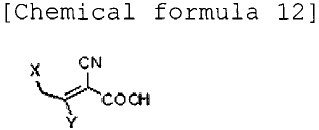

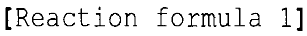

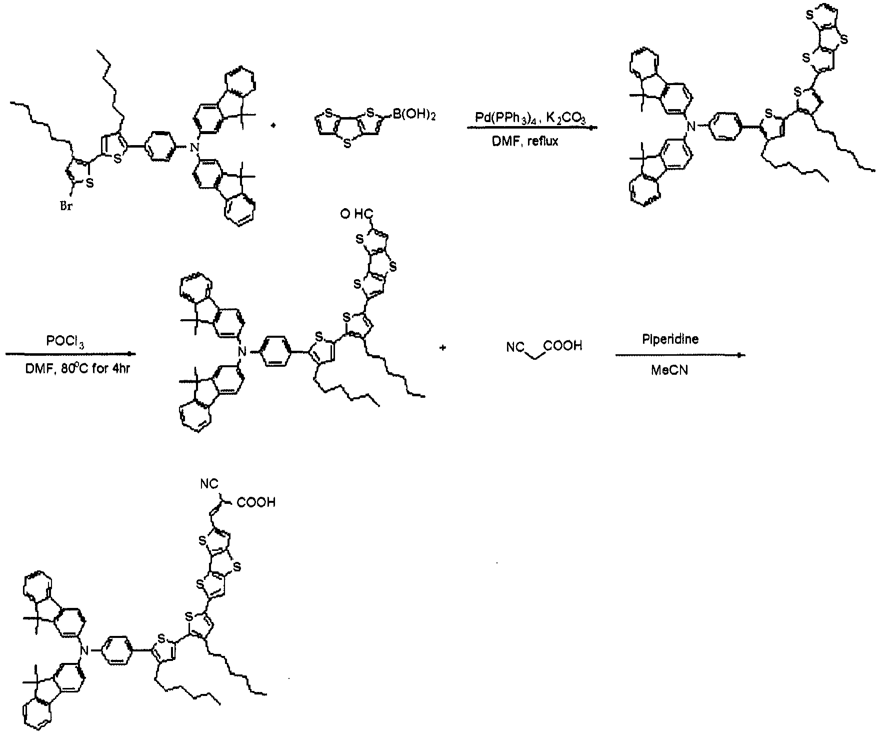

In the chemical formulas 8 to 12 and the reaction formula a, R₁ to R₃, X, Y and Ar are as defined in the chemical formula 7.

In the reaction formula, the compound in the chemical formula 8 which is used as a starting material in preparing the dye in the chemical formula 7 may be prepared by typical methods or may be purchased. Dithienothiophene which is a base of the compound in the chemical formula 9 may be prepared as in a reaction formula b below, which needs relatively short reaction time, and allows a very simple synthesis such as recrystallization without column chromatography (refer to [Chem. Mater. 2007, 19, 4007-4015] and [J. Mater. Chem. 1999, 9, 1719-1725]).

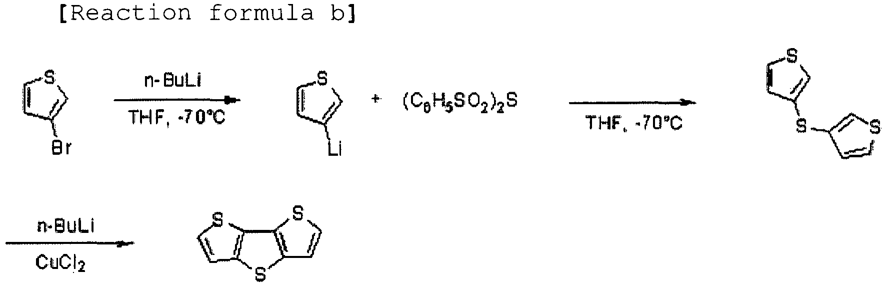

Further, the present invention provides a dye-sensitized photoelectric conversion element. The dye-sensitized photoelectric conversion element includes oxide semiconductor particles carrying the dye represented by one of the compounds 158 to 180. The dye-sensitized photoelectric conversion element according to the present invention may be prepared with conventional dyes to be included in a solar cell, as well as prepared with the dye represented by the one of the compounds 158 to 180. Preferably, the dye-sensitized photoelectric conversion element according to the present invention is prepared by forming an oxide semiconductor layer on a substrate with the oxide semiconductor particles and then by applying the dye according to the present invention to the thin film.

The substrate which has the oxide semiconductor particles layer according to the present invention preferably has a conductive surface. A commercial substrate may be used. More specifically, the substrate may include a glass or a transparent high molecular material such as polyethyleneterephthalate or polyethersulfone on which a conductive metal oxide layer such as tin oxide including indium, fluorine and antinomy or a metal layer such as steel, silver, gold and the like is formed. The conductivity is preferably 1000Ω and less, and more preferably, 100Ω and less.

The particles of the oxide semiconductor preferably include metal oxide. More specifically, the metal oxide may include titan, tin, zinc, tungsten, zirconium, gallium, indium, yttrium, niobium, tantalum, vanadium and the like. The metal oxide preferably includes titan, tin, zinc, niobium, indium and the like, and more preferably, titanium dioxide, zinc oxide and tin oxide and most preferably, titanium dioxide. The oxide semiconductor may be used alone, mixed with others or coated on a surface of a semiconductor.

A diameter of the oxide semiconductor particles is an average of 1 to 500nm, and more preferably, 1 to 100nm. The oxide semiconductor particles may include large and small diameters combined. Alternatively, the oxide semiconductor particles may include multiple layers.

The oxide semiconductor layer may be formed by spraying oxide semiconductor particles on a substrate, by electrically extracting a semiconductor particles layer from a substrate as an electrode or by applying a paste on a substrate to be dried, cured or fired. The paste includes particles which are created by hydrolyzing a precursor of semiconductor particles such as slurry of semiconductor particles or semiconductor alkoxide and the like. Preferably, the oxide semiconductor layer is formed by applying the paste on the substrate. In this case, the secondary-coagulated oxide semiconductor particles are dispersed in a dispersion medium by a known method to have an average primary diameter of 1 to 200nm to thereby form the slurry.

The dispersion medium which disperses the slurry may vary as long as it disperses the semiconductor particles. More specifically, the dispersion medium may include water, alcohol such as ethanol, ketone such as acetone, acetyl acetone or hydrocarbon such as hexane, which may be mixed and used. Preferably, the dispersion medium includes water since it less changes viscosity of the slurry. A dispersion stabilizer may be used to stabilize dispersion of the oxide semiconductor particles. More specifically, the dispersion stabilizer may include e.g., acid such as acetic acid, hydrochloric acid and nitric acid, acetyl acetone, acrylic acid, polyethyleneglycol, polyvinylalcohol and the like.

The substrate which is applied with the slurry may be fired at firing temperatures of 100°C and above, and preferably, 200°C and above. The upper limit of the firing temperatures is the melting point (softening point) of the material, i.e., 900°C, and preferably, 600°C and below. The firing temperatures according to the present invention are not limited particularly, but preferably within 4 hours.

According to the present invention, a thickness of the layer on the substrate may be 1 to 200*µ*m, and preferably, 1 to 50*µ*m. If the substrate is fired, some of the oxide semiconductor particles layer are melted and attached, but do not affect the present invention particularly.

The oxide semiconductor layer may be secondary treated. For example, the layer may be applied with a solution of alkoxide, chloride, nitride and sulfide which are the same metal as a semiconductor, and then dried or fired again to improve performance. The metal alkoxide may include titanethoxide, titaniumisoproepoxide, titan t-butoxide, n-dibutyldiacetyl tin and the like, or an alcohol solution thereof. The chloride may include e.g., titanium tetrachloride, tin tetrachloride, zinc chloride and the like, or an aqueous solution thereof. The obtained oxide semiconductor layer includes particles of the oxide semiconductor.

A method of applying the dye to the oxide semiconductor particles layer according to the present invention is not limited to a particular method. More specifically, the substrate having the oxide semiconductor layer may be dipped into a solution which is made by dissolving the dye represented by the chemical formula 7 including a structure of compounds 158 to 160 with a solvent or dipped into a dispersion solution which is made by dispersing the dye. The concentration of the solution or the dispersion solution may be properly determined depending on the dye. The applying temperature ranges from normal temperatures to the boiling point of the solvent. The applying time ranges from one minute to 48 hours. More specifically, the solvent which dissolves the dye may include e.g., methanol, ethanol, acetonitrile, dimethylsulfoxide, dimethylformamide, acetone, t-butanol, etc. The dye concentration of the solution is typically 1X10-⁶M to 1M, and preferably, 1X10⁻⁵M to 1X10⁻¹M. Thus, the present invention may provide a dye-sensitized photoelectric conversion element which includes the oxide semiconductor particles layer.

The dye which is represented by the chemical formula 7 including a structure of compounds 158 to 180 may include a single or several dyes mixed. If the dyes are mixed, or other dyes or metal complex dyes may be mixed together. The metal complex dye to be mixed may include e.g., ruthenium complex or triarylmethylium salt thereof, phthalocyanine, porphyrin, etc., but not limited thereto. The organic dye to be mixed may include metal-free phthalocyanine, porphyrine, cyanine, merocyanine, oxonol, triphenylmethane, metin dye such as acrylic acid dye disclosed in WO2002/011213, xanthen dye, azo dye, anthraquinone dye, perylene dye and the like (refer to M. K. Nazeeruddin, A. Kay, I. Rodicio, R. Humphry-Baker, E. Muller, P. Liska, N. Vlachopoulos and M. Gratzel, J. Am. Chem. Soc., 1993, vol. 115, p. 6382). If at least two dyes are used, the dyes may sequentially be applied to the semiconductor layer, or mixed and melted together to be applied to the semiconductor layer.

If the oxide semiconductor particles layer according to the present invention is dipped into the dyes, the layer may be dipped into the dye under the presence of an inclusion compound to prevent the dyes from being bonded to one another. The inclusion compound may include cholic acid such as deoxycholic acid, dehydrodeoxycholic acid, chenodeoxycholic acid, cholic acid methylester, cholic acid natrium and the like, a steroid compound such as polethyleneoxide, cholic acid and the like, crown ether, cyclodextrin, calixarene, polyethyleneoxide, etc.

After being dipped into the dye, the electrode surface of the semiconductor layer may be treated by an amine compound such as 4-t-butyl pyridine or by a compound such as acetic acid, propionic acid which has an acidic group. For example, the substrate which has the semiconductor particles layer carrying the dye may be dipped into an ethanol solution of amine.

Further, the present invention provides a dye-sensitized solar cell which includes the dye-sensitized photoelectric conversion element. The solar cell may be manufactured by a known method by using a conventional photoelectric conversion element as well as using the dye-sensitized photoelectric conversion element having the oxide semiconductor particles layer carrying the dye represented by the chemical formula 7 including a structure of compounds 158 to 180. More specifically, the dye-sensitized solar cell may include an electrode (negative) of a photoelectric conversion element which is formed by applying the dye represented by the chemical formula 7 including a structure of compounds 158 to 180 to the oxide semiconductor particles layer, a counter electrode (positive), a redox electrolyte, a hole transferring material or a p-type semiconductor.

Preferably, the preparation of the dye-sensitized solar cell according to the present invention includes an operation of coating TiO₂ paste on a transparent conductive substrate, an operation of firing the paste-coated substrate to form a TiO₂ layer, an operation of dipping the substrate having the TiO₂ layer into a solution including the dissolved dye represented by the chemical formula 7 including a structure of compounds 158 to 180 to form a TiO₂ film electrode having the dye, an operation of providing a second glass substrate including a counter electrode on the TiO₂ film electrode, an operation of forming a hole to pass through the second glass substrate and the counter electrode, an operation of coupling the counter electrode and the TiO₂ film electrode by heat and pressure, leaving a thermoplastic polymer film therebetween, an operation of injecting an electrolyte to the thermoplastic polymer film interposed between the counter electrode and the TiO₂ film electrode through the hole and an operation of sealing the thermoplastic polymer film.

The redox electrolyte, the hole transferring material and the p-type semiconductor may be liquid, coagulate (gel and gel phase), solid, etc. The redox electrolyte, the dissolved salt, the hole transferring material and the p-type semiconductor may be liquid by being dissolved by a solvent or may include normal temperature-molten salt. The redox electrolyte, the hole transferring material and the p-type semiconductor may be coagulate (gel and gel phase) by being included in a polymer matrix or a monomer gelation agent. The redox electrolyte, the dissolved salt, the hole transferring material and the p-type semiconductor may be solid.

The hole transferring material may include an amine derivative, a conductive polymer such as polyacetylene, polyaniline, polythiophene, a material such as a triphenylene compound using a discotheque liquid crystal phase, etc. The p-type semiconductor may include CuI, CuSCN and the like. Preferably, the counter electrode is conductive and acts as a catalyst for a reduction reaction of the redox electrolyte. For example, platinum, carbon, rhodium or ruthenium may be deposited on a glass or a polymer film, or conductive particles may be applied to the glass or the polymer film to form the counter electrode.

The redox electrolyte of the solar cell according to the present invention may include a halogen oxidation reduction electrolyte including a halogen compound having a halogen ion as a counter ion and a halogen molecule, a metal redox electrolyte such as ferrocyanide-ferrocyanide, ferrocene-ferricinium ion or a metal complex such as a cobalt complex, an organic oxidation reduction electrolyte such as alkylthiol-alkyldisulfide, viologen dye and hydroquinone-quinone and the like. Preferably, the redox electrolyte includes a halogen oxidation reduction electrolyte. Preferably, the halogen molecule of the halogen oxidation reduction electrolyte including the halogen compound-halogen molecule includes an iodine molecule. The halogen compound which has a halogen ion as a counter ion may include halogenated metal salt such as LiI, NaI, KI, CaI₂, MgI₂, CuI, halogen organic ammonium salt such as tetraalkylammoniumiodine, imidazoliumiodine, phyridiumiodine, or I₂.

The redox electrolyte may include a solution added with the foregoing materials. In this case, a solvent may electrochemically be inactive. More specifically, the solvent may include e.g., acetonitrile, propylenecarbonate, ethylenecarbonate, 3-methoxypropionitrile, methoxyacetonitrile, ethyleneglycol, propyleneglycol, diethyleneglycol, triethyleneglycol, butylolactone, dimethoxyethane, dimethylcarbonate, 1,3-dioxolane, methylformate, 2-methyltetrahydrofurane, 3-methoxy-oxazolidine-2-on, sulforane, tetrahydrofurane, water and the like. Preferably, the solvent includes acetonitrile, propylenecarbonate, ethylenecarbonate, 3-methoxypropionitrile, ethyleneglycol, 3-methoxy-oxazolidine-2-on, butylolactone, etc. The solvent may be used alone or mixed together. The gel phase positive electrolyte may be formed by adding an electrolyte or an electrolyte solution to a matrix such as oligomer and polymer or by adding an electrolyte or an electrolyte solution to a monomer gelation agent. The concentration of the redox electrolyte may be 0.01 to 99wt%, and more preferably, 0.1 to 30wt%.

The solar cell according to the present invention may be manufactured by providing the photoelectric conversion element (negative) having the oxide semiconductor particles carrying the dye and the counter electrode (positive) facing the photoelectric conversion element and by injecting the solution including the redox electrolyte therebetween.

Hereinafter, exemplary embodiments of the present invention are provided to help understand the present invention. However, the present invention is not limited to following exemplary embodiments.

### [Exemplary embodiments]

### Synthesis of dye

All reactions were implemented under argon condition, and all solvents used were distilled by a proper reagent from Sigma-Aldrich. 1H NMR spectrum was measured by Varian Mercury 300 spectrometer. Absorbance and emission spectra were measured by Perkin-Elmer Lambda 2S UV-visible spectrophotometer and Perkin LS fluorescence spectrometer, respectively.

### [Exemplary embodiment 1] Synthesis of Compound 1 (not part of the invention)

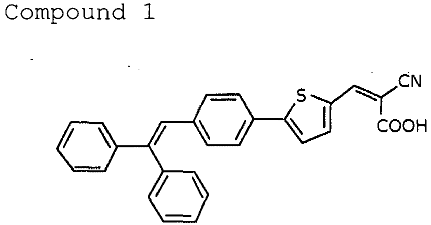

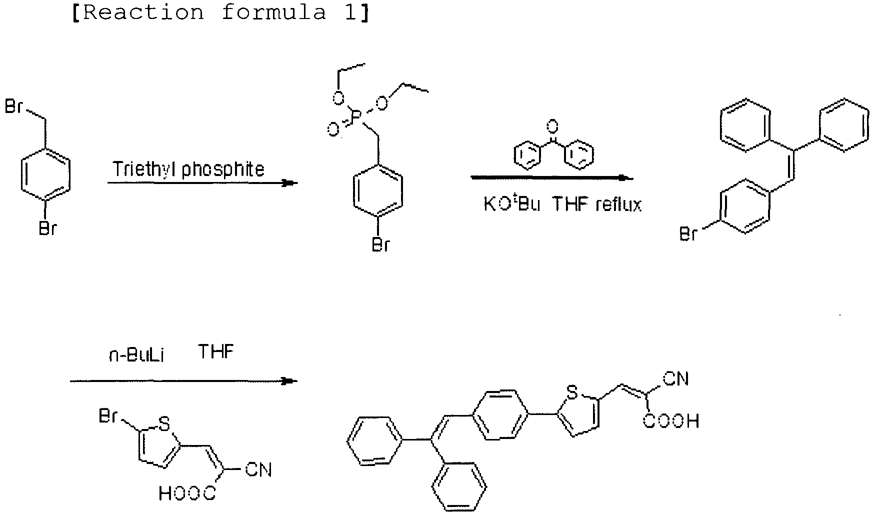

Together with triethylphosphite (25g, 150mmol), 1-bromo-4-bromomethylbenzene (5g, 20mmol) was put into an agitator to have them react to each other for two hours at 120°C to thereby synthesize triethyl 4-bromobenzylphosphite. The synthesized triethyl 4-bromobenzylphosphite (6.74g, 20mmol), benzophenone (5.13g, 29.16mmol) and potassium tert-butoxide (3.27g, 29.16mmol) were put into a reactor and melted by THF of 50ml to have them react to each other for four hours at 120°C to thereby synthesize 1-bromo-4-(2,2-diphenylvinyl)benzene. After melted by THF 50ml, 1-bromo-4-(2,2-diphenylvinyl)benzene (6.03g, 18mmol) was gradually added with 2Mn-BuLi (10ml, 20mmol) at -78°C to be agitated for one hour at low temperatures. Again, the mixture was gradually added with (Z)-3-(5-bromothiophene-2-yl)-2-cyanoacrylic acid (5.16g, 20mmol) at -78°C, agitated for one hour at low temperatures and then additionally agitated for 30 minutes at 0°C to complete the reaction.

After extracting an organic layer from the mixture with dichloromethane and distilled water, the solvent was removed through a distiller, the organic layer was recrystallized by n-hexane, and dried and purified after filtering sediment (yield 52%).

¹H NMR (CDCl₃) : [ppm] = 8.04 (s, 1H), 7.67 (s, 1H), 7.60 (d, ³J_{HH} = 3.6Hz, 2H), 7.52 (d, ³J_{HH} = 8.4Hz, 2H), 7.43 (m, 3H), 7.34 (m, 5H), 7.19 (dd, ³J_{HH} = 8Hz, 2H), 7.14 (s, 1H), 7.07 (d, ³J_{HH} = 8.4Hz, 2H).

### [Exemplary embodiment 2] Synthesis of compound 3 (not part of the invention)

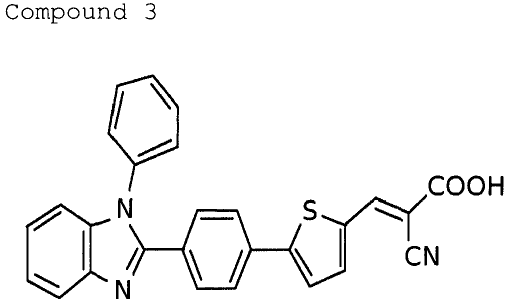

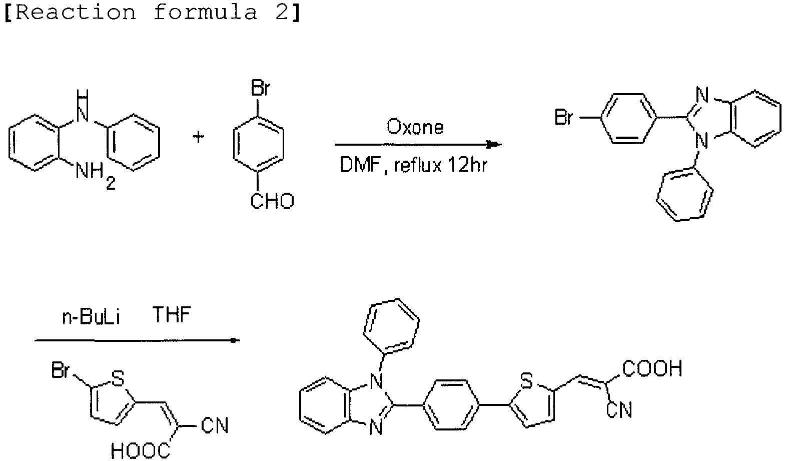

4-bromobenzaldehyde (5g, 27mmol), N1-phenylbenzene-1,2-diamine (5.47g, 29.7mmol) and oxone (18.25g, 29.7mmol) were put into a reactor, melted by DMF of 80ml and agitated for 10 hours at 120°C to thereby synthesize 2-(4-bromophenyl)-1-phenyl-1H-benzo[d]imidazole. The synthesized 2-(4-bromophenyl)-1-phenyl-1H-benzo[d]imidazole (6.98g, 20mmol) was melted by THF of 50ml, gradually added with 2Mn-BuLi (11ml, 22mmol) at - 78°C and agitated for one hour at low temperatures. The mixture was added with (Z)-3-(5-bromothiophene-2-yl)-2-cyanoacrylic acid (5.16g, 20mmol) at -78°C, agitated for one hour at low temperatures and then additionally agitated for 30 minutes at 0°C to complete the reaction.

After extracting an organic layer from the mixture with dichloromethane and distilled water, the solvent was removed through a distiller, the organic layer was recrystallized by n-hexane, and dried and purified after filtering sediment (yield 48%).

¹H NMR(CDCl₃) : 11.0 (s, 1H), 8.16 (s, 1H), 7.70 (m, 3H), 7.54 (dd, ³J_{HH} = 8Hz, 4H), 7.3 (s, 1H), 7.26 (m, 7H).

### [Exemplary embodiment 3] Synthesis of compound 6 (not part of the invention)

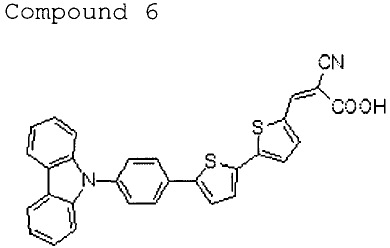

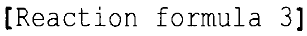

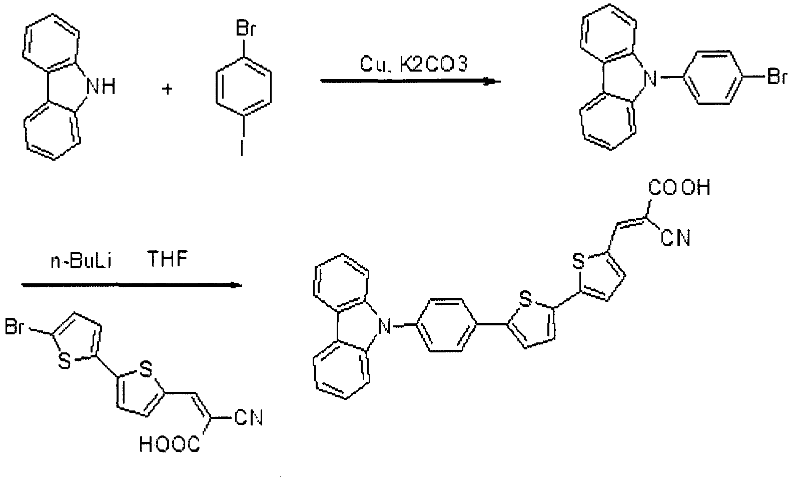

1-bromo-4-iodobenzene (5g, 17.67mmol), carbazole (2.9g, 17.67mmol), Cu(0.06g, 0.88mmol) and K₂CO₃ (4.88g, 35.34mmol) were melted by toluene of 30ml and agitated for 10 hours at 120°C to thereby synthesize 9-(4-bromophenyl)-9H-carbazole. The synthesized 9-(4-bromophenyl)-9H-carbazole(4.83g, 15mmol) was melted by THF of 50ml, gradually added with 2Mn-BuLi (7.5ml, 15mmol) at -78°C and agitated for one hour at low temperatures. The mixture was added with 3-(5'-bromo-[2,2']bithiophene-5-yl)-2-cyanoacrylic acid (5.44g, 16mmol) at -78°C, agitated for one hour at low temperatures and then additionally agitated for 30 minutes at 0°C to complete the reaction.

After extracting an organic layer from the mixture with dichloromethane and distilled water, the solvent was removed through a distiller, the organic layer was recrystallized by n-hexane, and dried and purified after filtering sediment (yield 60%).

¹H NMR(CDCl₃) : 8.26 (d, ³J_{HH} = 8Hz, 2H), 8.01 (m, 3H), 7. 68 (m, 4H), 7. 57 (d, ³J_{HH} = 4Hz, 1H), 7. 51 (d, ³J_{HH} = 4Hz, 1H) 7.46 (m, 5H), 7.28 (m, 2H).

### [Exemplary embodiment 4] Synthesis of compound 8 (not part of the invention)

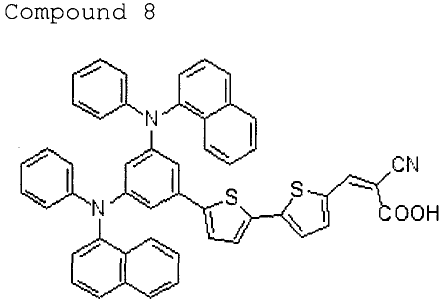

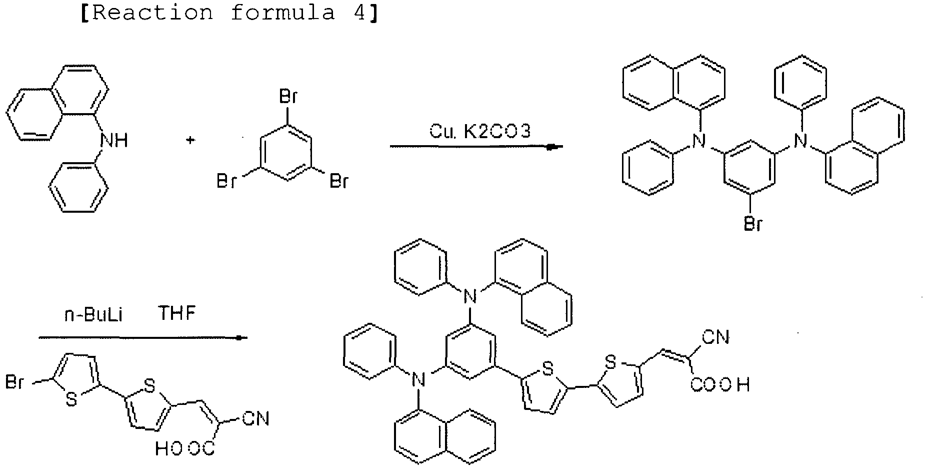

1,3,5-tribromobenzene (2g, 6.35mmol), N-phenylnaphthalene-1-amine (2.79g, 12.7mmol), Cu(0.02g, 0.31mmol) and K₂CO₃ (1.75g, 12.7mmol) were melted by toluene of 30ml and agitated for 10 hours at 120°C to thereby synthesize 5-bromo-N1,N3-di(naphthalene-1-yl)-N1,N3-diphenylbenzene-1,3-diamine. The synthesized 5-bromo-N1,N3-di(naphthalene-1-yl)-N1,N3-diphenylbenzene-1,3-diamine (2.95g, 5mmol) was melted by THF of 40ml, gradually added with 2Mn-BuLi (7.5ml, 15mmol) at -78°C and agitated for one hour at low temperatures. The mixture was gradually added with (Z)-3-(5-bromothiophene-2-yl)-2-cyanoacrylic acid (5.44g, 16mmol) at -78°C, agitated for one hour at low temperatures and then additionally agitated for 30 minutes at 0°C to complete the reaction.

After extracting an organic layer from the mixture with dichloromethane and distilled water, the solvent was removed through a distiller, the organic layer was recrystallized by n-hexane, and dried and purified after filtering sediment (yield 57%).

¹H NMR (CDCl₃) : 7.98 (m, 3H), 7.85 (d, ³J_{HH} = 8Hz, 2H), 7.79 (d, ³J_{HH} = 8Hz, 2H), 7.42 (m, 8H), 7.33 (m, 3H), 7.26 (d, ³J_{HH} = 4Hz, 1H), 7.10 (m, 4H), 6. 92 (d, ³J_{HH} = 4Hz, 1H), 6.87 (m, 7H), 6.58 (d, ³J_{HH} = 2Hz, 1H), 6.41 (s, 1H)

### [Exemplary embodiments 5 to 7] Synthesis of compounds 2, 4 and 5 (not part of the invention)

Compounds 2, 4 and 5 were prepared by a method similar to those of the exemplary embodiments 1 to 3.

### [Exemplary embodiment 8] Synthesis of compound 142 (not part of the invention)

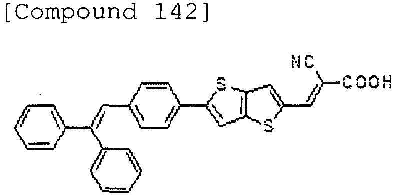

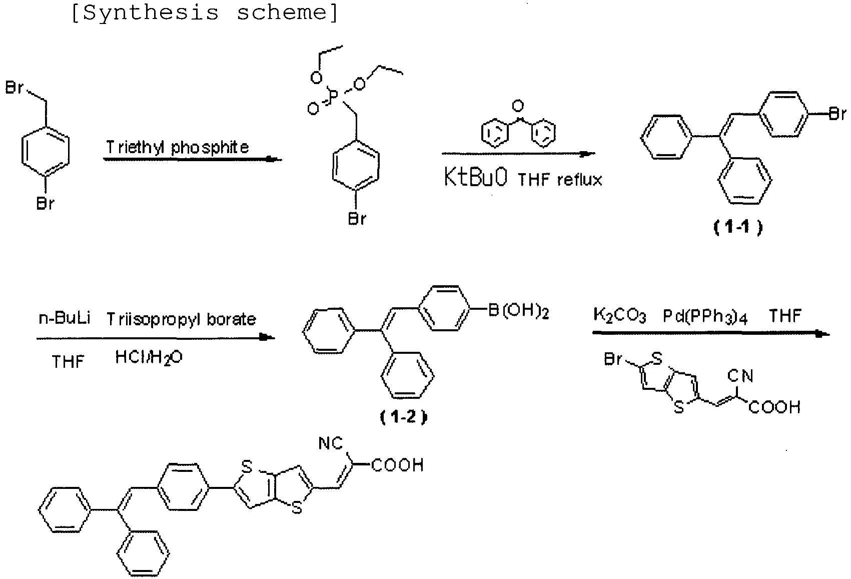

Together with triethylphosphite (25g, 150mmol), 1-bromo-4-bromomethylbenzene (5g, 20mmol) was put into an agitator to have them react to each other for two hours at 120°C to thereby synthesize triethyl 4-bromobenzylphosphite. The synthesized triethyl 4-bromobenzylphosphite (6.74g, 20mmol), benzophenone (5.13g, 29.16mmol) and potassium tert-butoxide (3.27g, 29.16mmol) were put into a reactor and melted by THF of 50ml to have them react to each other for four hours at 120°C to thereby synthesize 1-bromo-4-(2,2-diphenylvinyl)benzene(1-1). The synthesized-bromo-4-(2,2-diphenylvinyl)benzene(1-1) (6.03g, 18mmol) was melted by THF of 50ml, gradually added with 2Mn-BuLi (10ml, 20mmol) at -78°C and agitated for one hour at low temperatures. The mixture was gradually added with triisopropyl borate (3.76g, 20mmol) at -78°C, agitated for one hour at low temperatures and then additionally agitated for 30 minutes at 0°C to thereby synthesize 4-(2,2-diphenylvinyl)phenylboronic acid (1-2). The synthesized 4-(2,2-diphenylvinyl)phenylboronic acid (4.5g, 15mmol)(1-2), K₂CO₃ (6.2g, 45mmol) and Pd(PPh₃)₄(0.86g, 0.75mmol) were melted by THF (60ml), added with (E)-3-(5-bromothieno[3,2-b]thiophene-2-yl)-2-cyanoacrylic acid (5.6g, 18mmol), agitated for four hours at 120°C to complete the reaction. After extracting an organic layer from the mixture with dichloromethane and distilled water, the solvent was removed through an evaporator and the organic layer was recrystallized by n-hexane and dried to collect the compound 142 after the sediment was filtered.

### [Exemplary embodiment 9] Synthesis of compound 144 (not part of the invention)

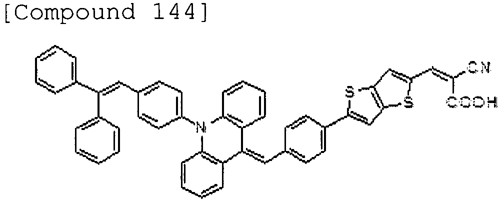

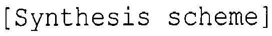

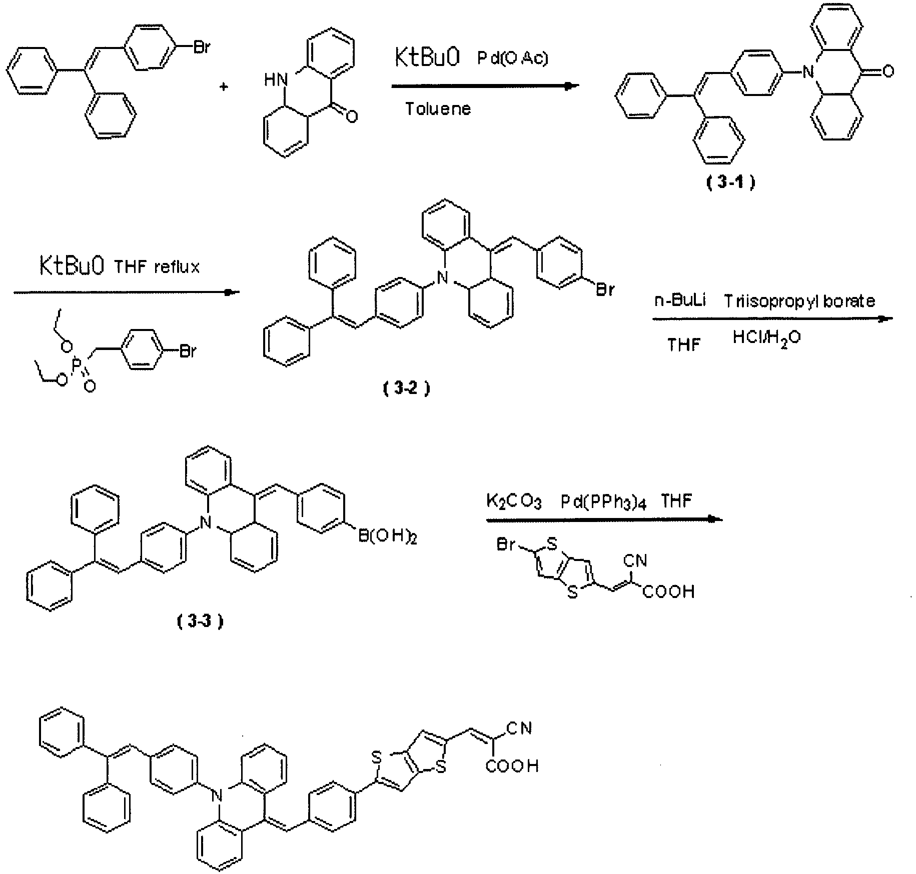

1-bromo-4-(2,2-diphenylvinyl)benzene (6.03g, 18mmol) and 10,,10,,-dihydroacridin-9(8aH)-one(4.26g, 21.6mmol) were put into a reaction container, added with KtBuO(4.84g, 43.2mmol) and Pd(OAc)(0.24g, 1.08mmol) and melted by toluene (50ml) to have them react to each other for 12 hours at 150°C to thereby synthesize a compound 10-(4-(2,2-diphenylvinyl)phenyl)-10,,10,,-dihydroacridin-9(8aH)-one(3-1). The synthesized compound 10-(4-(2,2-diphenylvinyl)phenyl)-10,,10,,-dihydroacridin-9(8aH)-one(3-1)(3.2g, 7.08mmol) and triethyl 4-bromobenzylphosphite (2.6g, 8.49mmol) were put into a reactor, added with KtBuO (0.95g, 8.49mmol), melted by THF (50ml) and agitated for four hours at 120°C to synthesize a compound (E)-9-(4-bromobenzylidene)-10-(4-(2,2-diphenylvinyl)phenyl)-8a,9,10,,10,,-tetrahydroacridine(3-2). The synthesized compound (E)-9-(4-bromobenzylidene)-10-(4-(2,2-diphenylvinyl)phenyl)-8a,9,10,,10,,-tetrahydroacridine(3-2) (3.5g, 5.78mmol) was melted by THF of 30ml, gradually added with 2Mn-BuLi (3.45g, 6.9mmol) at -78°C and agitated for one hour at low temperatures. The mixture was gradually added with triisoprophyl borate (1.29g, 6.9mmol) at -78°C, agitated for one hour at low temperatures and then additionally agitated for 30 minutes at 0°C to thereby synthesize a compound (E)-4-((10-(4-(2,2-diphenylvinyl)phenyl)-10,,10,,-dihydroacridin-9(8aH)-ylidene)methyl)phenylboronic acid (3-3). The synthesized compound (E)-4-((10-(4-(2,2-diphenylvinyl)phenyl)-10,,10,,-dihydroacridin-9(8aH)-ylidene)methyl)phenylboronic acid (3-3) (2.8g, 4.9mmol), K₂CO₃(2.03g, 14.7mmol) and Pd(PPh₃)₄(0.86g, 0.29mmol) were melted by THF (30ml), added with (E)-3-(5-bromothieno[3,2-b]thiophene-2-yl)-2-cyanoacrylic acid (5.6g, 18mmol) and agitated for four hours at 120°C to complete the reaction. After extracting an organic layer from the mixture with dichloromethane and distilled water, the solvent was removed through an evaporator and the organic layer was recrystallized by n-hexane, and dried to collect the compound 144 after the sediment was filtered.

### [Exemplary embodiment 10] Synthesis of compound 146 (not part of the invention)

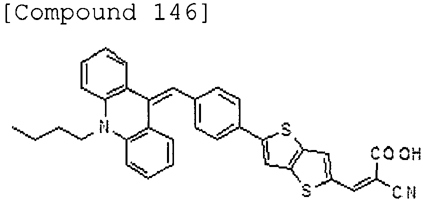

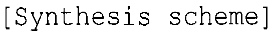

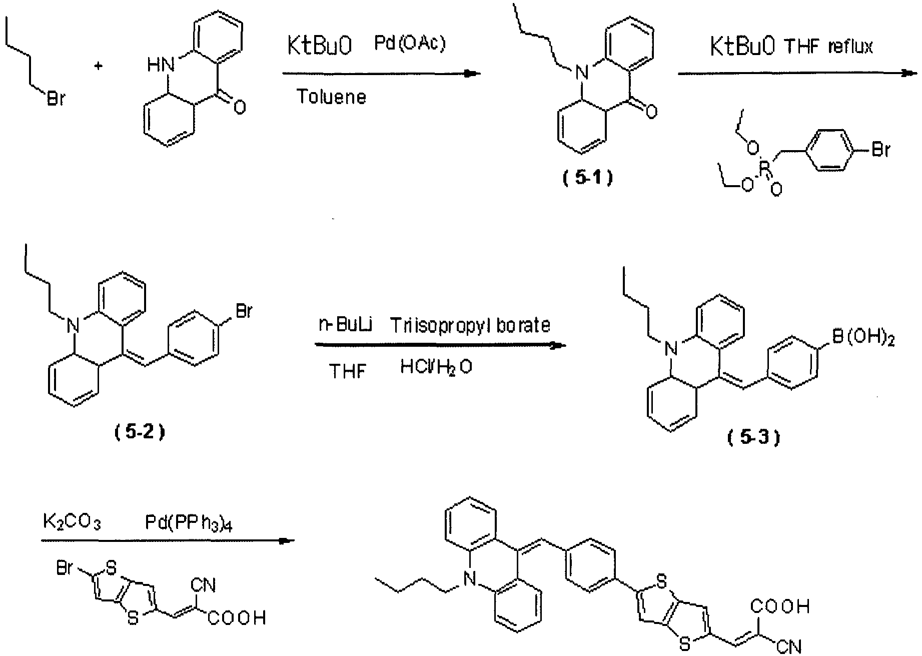

1-bromobutane (5g, 36.5mmol) and 10,,10,,-dihydroacridin-9(8aH)-one(7.19g, 36.5mmol) were put into a reactor, added with KtBuO(9.8g, 87.6mmol) and Pd(OAc)(0.49g, 2.19mmol) and melted by toluene (60ml) to have them react to one another for 12 hours at 150°C to thereby synthesize a compound 10-butyl-10,,10,,-dihydroacridin-9(8aH)-one(5-1). The synthesized compound 10-butyl-10,,10,,-dihydroacridin-9(8aH)-one(5-1) (4.2g, 16.5mmol) and triethyl 4-bromobenzylphosphite (6.08g, 19.8mmol) were put into a reactor, added with KtBuO (2.22g, 19.8mmol), melted by THF (50ml) and agitated for four hours at 120°C to thereby synthesize a compound (Z)-9-(4-bromobenzylidene)-10-butyl-8a,9,10,,10,,-tetrahydroacridine(5-2). The synthesized compound (Z)-9-(4-bromobenzylidene)-10-butyl-8a, 9,10,,10,,-tetrahydroacridine(5-2) (6.09g, 15mmol) was melted by THF of 50ml, gradually added with Mn-BuLi (9ml, 18mmol) at - 78°C and agitated for one hour at low temperatures. The mixture was gradually added with triisopropyl borate (3.38g, 18mmol) at -78°C, agitated for one hour at low temperatures and then additionally agitated for 30 minutes at 0°C to thereby synthesize a compound (Z)-4-((10-butyl-10,,10,,dihydroacridin-9(8aH)-ylidene)methyl)phenylboronic acid (5-3). The synthesized compound (Z)-4-((10-butyl-10,,10,,dihydroacridin-9(8aH)-ylidene)methyl)phenylboronic acid (5-3)(4.82g, 13mmol), K₂CO₃(5.39g, 39mmol) and Pd(PPh₃)₄(0.99g, 0.65mmol) were melted by THF (30ml), added with (E)-3-(5-bromothieno[3,2-b]thiophene-2-yl)-2-cyanoacrylic acid (4.08g, 13mmol) and agitated for four hours at 120°C to complete the reaction. After extracting an organic layer from the mixture with dichloromethane and distilled water, the solvent was removed through an evaporator and the organic layer was recrystallized by n-hexane and dried to collect the compound 146 after the sediment was filtered. [Exemplary embodiment 11] Synthesis of compound 147

### (not part of the invention)

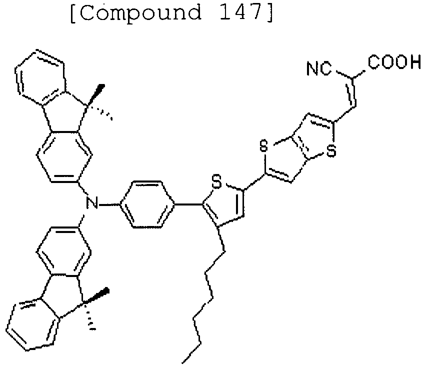

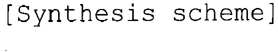

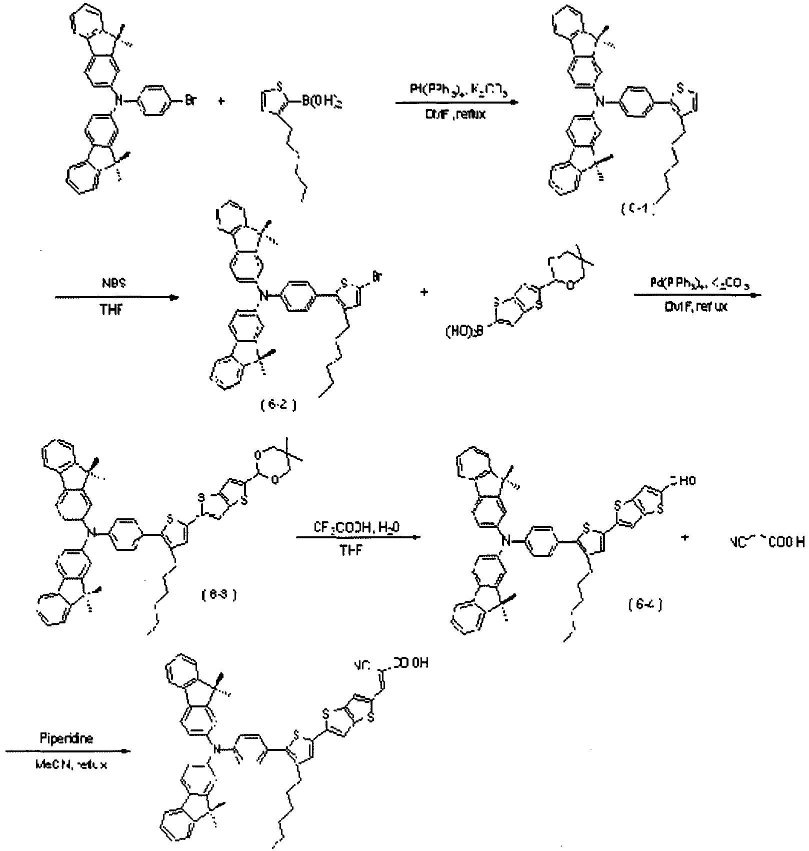

(4-bromo-phenyl)-bis-(9,9-dimethyl-9H-fluoren-2-yl)-amine (0.98g, 1.76mmol), 3-hexylthiophene-2-boronic acid (0.37g, 1.76mmol), tetrakis-(triphenylphosphine)palladium (0) (0.1g, 0.088mmol) and potassium carbonate (0.73g, 5.28mmol) were mixed, melted by DMF (40ml) and refluxed and agitated for 12 hours under nitrogen gas.

After the agitation, an organic layer was extracted from the mixture by using methylenechloride and water, evaporated and went through a column chromatography to synthesize N-(9,9-dimethyl-9H-fluoren-2-yl)-N-(4-(3-hexylthiophene-2-yl)phenyl)-9,9-dim-ethyl-9H-fluoren-2-amine **(6-1).** (eluent. M.C : Hx = 1:5) 1H NMR(CDCl₃):[ppm] = 0.89(m, 3H), 1.29(m, 4H), 1.52(m, 4H), 2.52(m, 2H), 1.47(s, 12H), 6.7(d, ³J_{HH} = 2.4Hz, 1H), 6.91(d, ³J_{HH} = 2.4Hz, 1H), 7.09(m, 4H), 7.31(m, 4H), 7.38(m, 4H), 7.58(d, ³J_{HH} = 11.6Hz, 2H), 7.62(m, 4H).

The synthesized N-(9,9-dimethyl-9H-fluoren-2-yl)-N-(4-(3-hexylthiophene-2-yl)phenyl)-9,9-dim-ethyl-9H-fluoren-2-amine **(6-1)** (0.91g, 1.42mmol) and NBS(0.3g, 1.7mmol) were melted by THF (30ml) and agitated for four hours under nitrogen atmosphere.

After the agitation, an organic layer was extracted from the mixture by using methylenechloride and water, evaporated and went through a column chromatography to synthesize N-(4-(5-bromo-3-hexylthiophene-2-yl)phenyl)-N-(9,9-dimethyl-9H-fluoren-2-yl)-9,9-dimethyl-9H-fluoren-2-amine (6-2). (eluent. M.C : Hx = 1:5).

The synthesized N-(4-(5-bromo-3-hexylthiophene-2-yl)phenyl)-N-(9,9-dimethyl-9H-fluoren-2-yl)-9,9-dimethyl-9H-fluoren-2-amine **(6-2)**(1.01g, 1.4mmol), 5-(5,5-dimethyl-1,3-dioxan-2-yl)thieno[3,2-b]thiophene-2-ylboronic acid (0.42g, 1.4mmol), tetrakis-(triphenylphosphine)palladium(0) (0.08g, 0.07mmol) and potassium carbonate (0.59g, 4.26mmol) were melted by THF (40ml) and refluxed and agitated for 12 hours under nitrogen gas.

After the agitation, an organic layer was extracted from the mixture by using methylenechloride and water, evaporated and went through a column chromatography to synthesize N-(4-(5-(5-(5,5-dimethyl-1,3-dioxan-2-yl)thieno[3,2-b]thiophene-2-yl)-3-hexy-1thiophene-2-yl)phenyl)-N-(9,9-dimethyl-9H-fluoren-2-yl)-9,9-dimethyl-9H-fluoren-2-amine **(6-3).** (eluent. M.C : Hx = 1:4).

The synthesized N-(4-(5-(5-(5,5-dimethyl-1,3-dioxan-2-yl)thieno[3,2-b]thiophene-2-yl)-3-hexy-1thiophene-2-yl)phenyl)-N-(9,9-dimethyl-9H-fluoren-2-yl)-9,9-dimethyl-9H-fluoren-2-amine **(6-3)**(1.07g, 1.2mol) was melted by THF (30ml), added with trifluoroacetic acid (0.26ml) and water (1ml) and agitated for four hours under nitrogen atmosphere.

After the agitation, an organic layer was extracted from the mixture by using methylenechloride and water, evaporated and went through a column chromatography to synthesize 5-(5-(4-(bis(9,9-dimethyl-9H-fluoren-2-yl)amino)phenyl)-4-hexylthiophene-2-yl)-thieno[3,2-b]thiophene-2-carbaldehyde(6-4). (eluent. M.C:Hx = 1:1).

The synthesized 5-(5-(4-(bis(9,9-dimethyl-9H-fluoren-2-yl)amino)phenyl)-4-hexylthiophene-2-yl)-thieno[3,2-b]thiophene-2-carbaldehyde(6-4)(0.89g, 1.0mmol), cyanoacetic acid (0.1g, 1.2mmol) and piperidine (0.12ml, 1.2mmol) were melted by acetonitrile (30ml) and refluxed and agitated for four hours.

After the agitation, an organic layer was extracted from the mixture by using methylenechloride and water, evaporated and went through a column chromatography to synthesize a compound 147. (eluent. EA:MeOH = 10:1)

### [Exemplary embodiment 12] Synthesis of Compound 148 (not part of the invention)

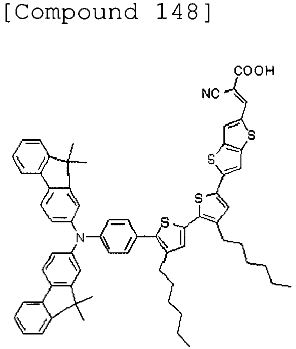

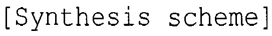

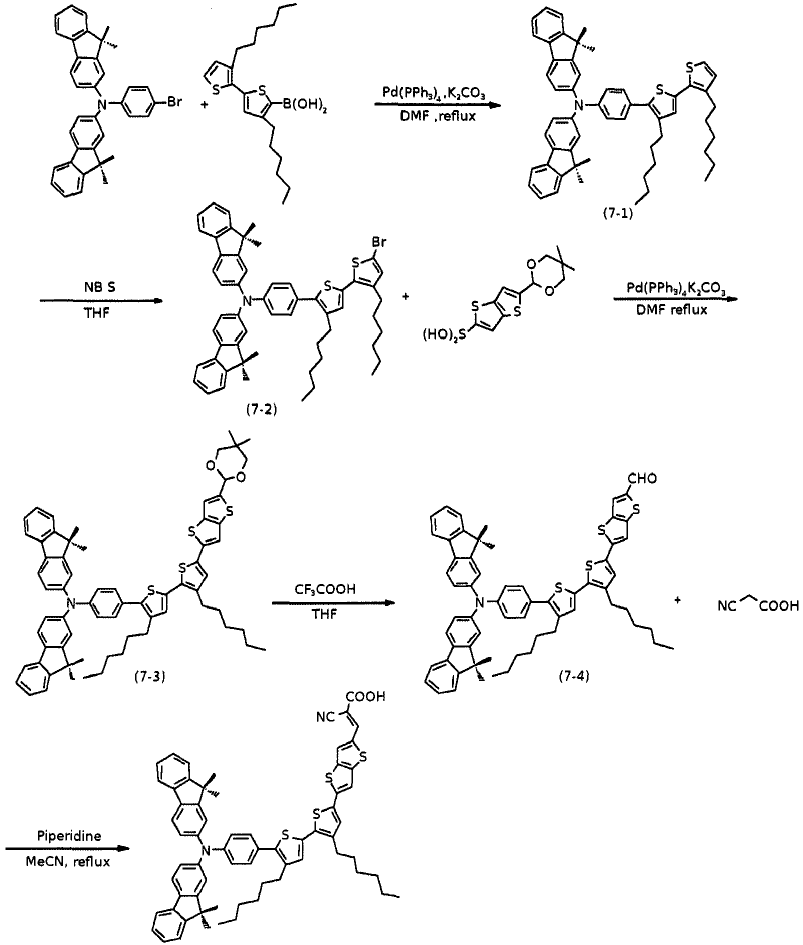

(4-bromo-phenyl)-bis-(9,9-dimethyl-9H-fluoren-2-yl)-amine (0.98g, 1.76mmol), 3',4-dihexyl-2,2'-bithiophene-5-ylboronic acid (0.66g, 1.76mmol), tetrakis-(tripheneylphos-phine)palladium(0) (0.1g, 0.088mmol) and potassium carbonate (0.73g, 5.28mmol) were melted by DMF (40ml) to synthesize a compound 148 with the same method as that in the exemplary embodiment 11.

### [Exemplary embodiment 13] Synthesis of a compound of chemical formula 149 (not part of the invention)

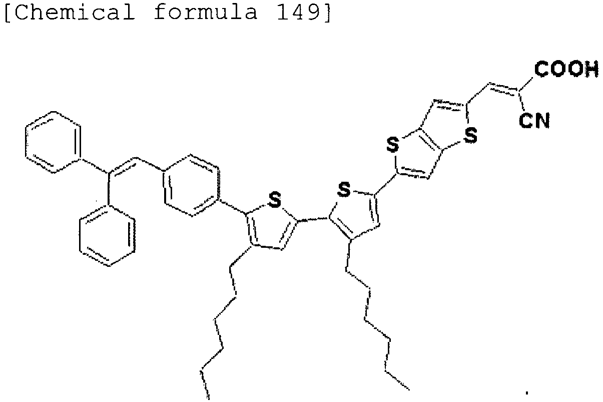

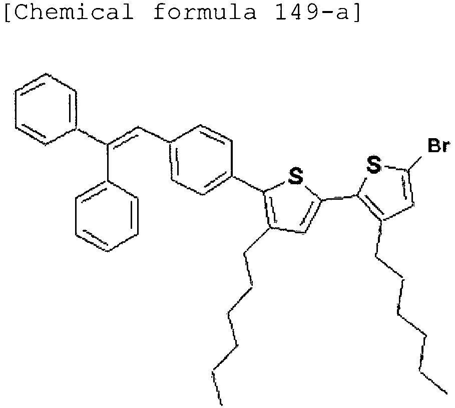

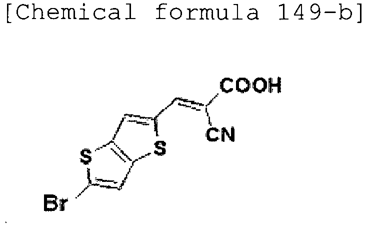

A compound in the chemical formula 149-a was melted by tetrahydrofurane (THF) and reacted to n-butyllithium and trialkyl borate consecutively at 0 to -100°C. The obtained reaction product reacted to a compound in the chemical formula 149-b in THF at 100 to 150°C to generate a compound in the chemical formula 149.

### [Exemplary embodiment 14] Synthesis of a compound of chemical formula 150 (not part of the invention)

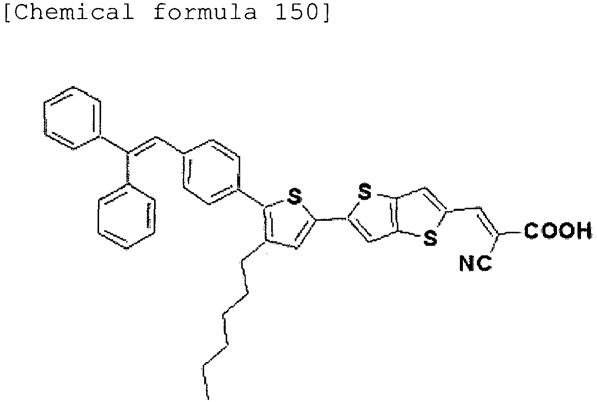

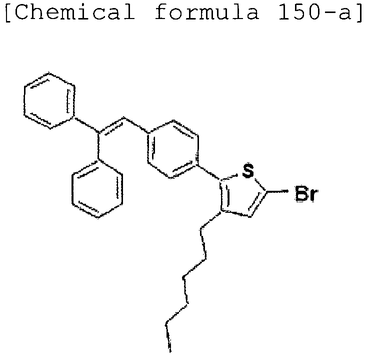

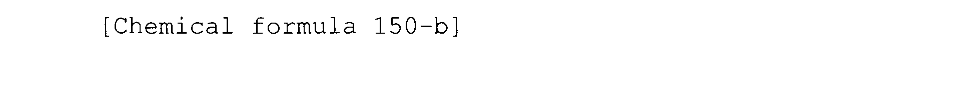

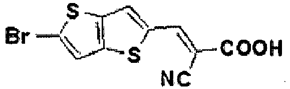

A compound in the chemical formula 150-a was melted by tetrahydrofurane (THF) and reacted to n-butyllithium and trialkyl borate consecutively at 0 to -100°C. The obtained reaction product reacted to a compound in the chemical formula 150-b in THF at 100 to 150°C to generate a compound in the chemical formula 150.

### [Exemplary embodiment 15] Synthesis of compound 154 (not part of the invention)

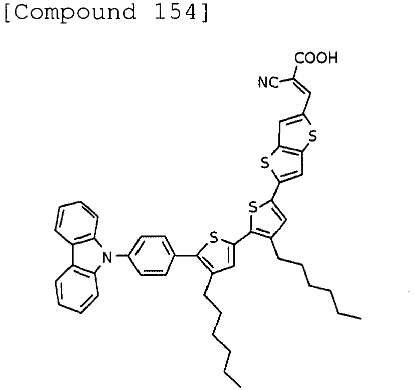

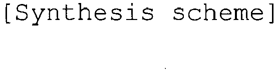

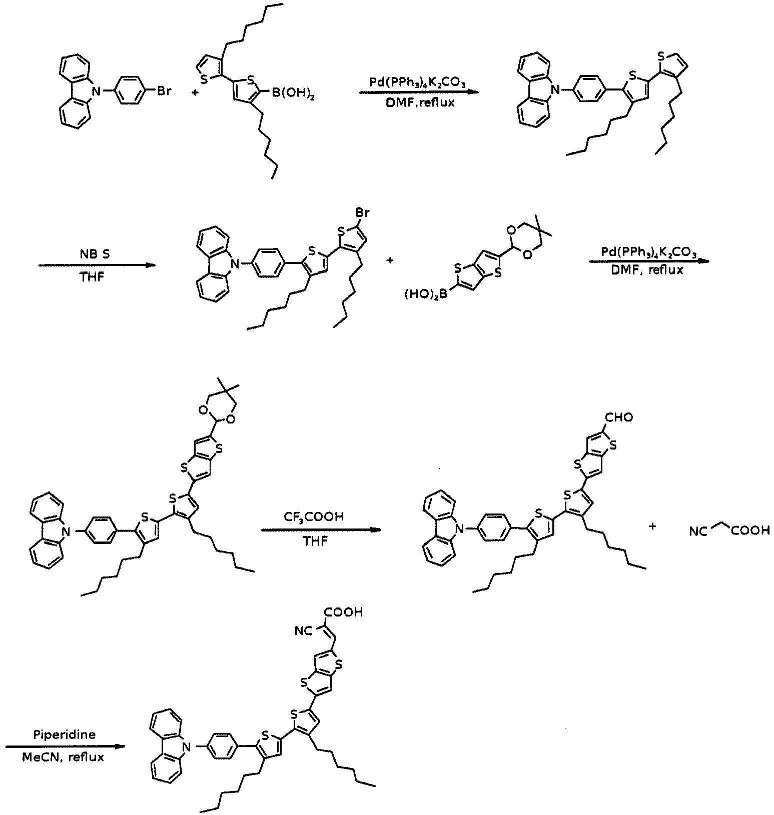

9-(4-bromophenyl)-9H-carbazole (0.56g, 1.76mmol), 3',4-dihexyl-2,2'-bithiophene-5-ylboronic acid (0.66g, 1.76mmol), tetrakis-(tripheneylphos-phine)palladium(0) (0.1g, 0.088mmol) and potassium carbonate (0.73g, 5.28mmol) were melted by DMF (40ml) to synthesize a compound 154 with the same method as that in the exemplary embodiment 11.

### [Exemplary embodiment 16] Synthesis of compound 157 (not part of the invention)

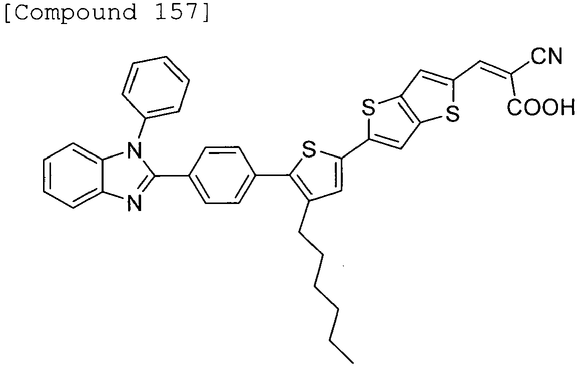

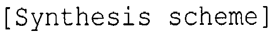

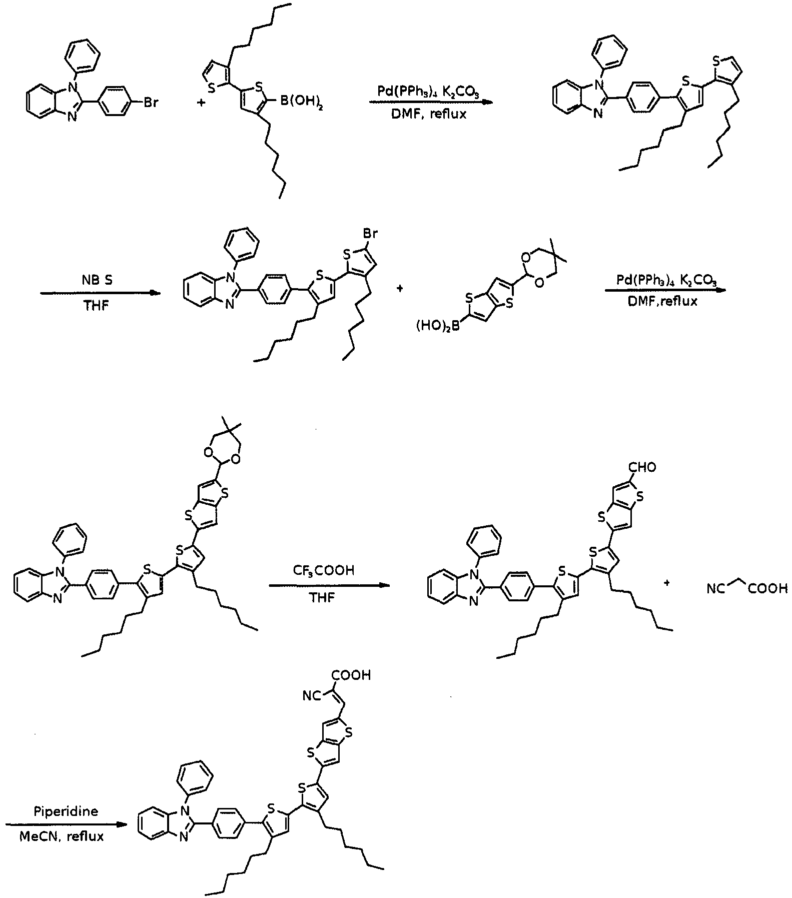

2-(4-bromophenyl)-1-phenyl-1H-benzo[d]imidazole (0.62g, 1.76mmol), 3',4-dihexyl-2,2'-bithiophen-5-ylboronic acid (0.66g, 1.76mmol), tetrakis-(tripheneylphos-phine)palladium(0) (0.1g, 0.088mmol) and potassium carbonate (0.73g, 5.28mmol) were melted by DMF (40ml) to synthesize a compound 157 with the same method as that in the exemplary embodiment 11.

### [Exemplary embodiment 17] Synthesis of compound 161

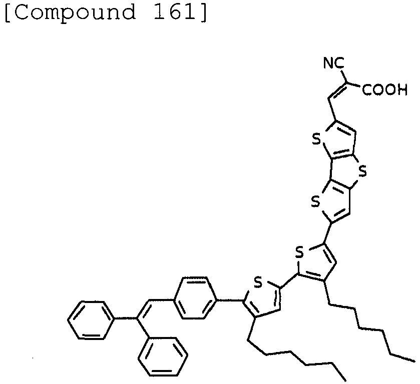

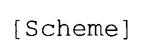

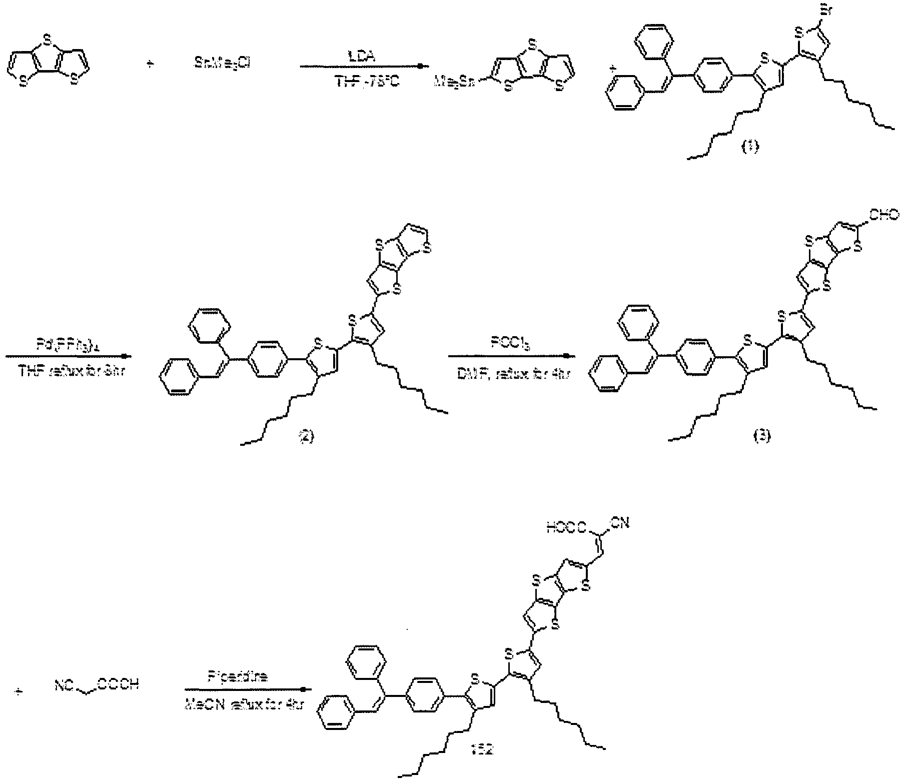

(1). Synthesized dithieno[2',3']thiophene (7g, 35.65mmol) was melted by THF (50ml), gradually added with LDA 2M (39ml) at -78°C, agitated for one hour at low temperatures, gradually added with trimethyltin chloride 1M (38ml) at -78°C, agitated for one hour at low temperatures and additionally agitated for 30 minutes at 0°C. After the agitation, an organic layer was extracted from the mixture by using methylenechloride and water, evaporated and dried. 1H NMR(CDCl₃) : [ppm] = 0.58 (s, 9H), 6. 98 (d, ³J_{HH} = 4.8Hz, 1H), 7.10 (s, 1H), 7.18 ((d, ³J_{HH} = 4.8Hz, 1H).
(2). 2-trimethyl(dithieno[2',3']thiophene-2-yl)stannane (0.48g, 1.34mmol), the compound (1) (0.74g, 1.12mmol), and Pd(PPh₃)₄ (0.065g, 0.056mmol) were melted by THF (40ml) and refluxed for eight hours under nitrogen atmosphere. Then, an organic layer was extracted from the mixture by using methylenechloride and water, evaporated and went through column chromatography. (eluent. M.C:Hx = 1:5) 1H NMR(CDCl₃) : [ppm] = 0.96 (m, 6H), 1.29 (m, 4H), 1.96 (m, 4H), 2.55 (m, 8H), 2.62 (m, 4H), 6.7 (m, 2H), 6.91 (m, 2H), 6.96 (d, ³J_{HH} = 4.8Hz, 1H), 7.08 (d, ³J_{HH} = 4.8Hz, 1H), 7.18 (m, 4H), 7.28 (m, 4H), 7.55 (m, 4H), 7.78 (d, ³J_{HH} = 8.8Hz, 2H).
(3). The compound (2)(0.09g, 1.2mmol) was melted by DMF (20ml), gradually added with phosphorous oxychloride (0.13ml, 1.44mmol) at 0°C and agitated for four hours at 80°C. After the agitation, an organic layer was extracted from the mixture by using methylenechloride and water, evaporated and went through column chromatography. (eluent. M.C : Hx = 1:4) 1H NMR(CDCl₃) : [ppm] = 0.96 (m, 6H), 1.29 (m, 4H), 1.96 (m, 4H), 2.55 (m, 8H), 2.62 (m, 4H), 6.7 (m, 2H), 6.91 (m, 2H), 7.08 (s, 1H), 7.18 (m, 4H), 7.28 (m, 4H), 7.55 (m, 4H), 7.78 (d, ³J_{HH} = 8.8Hz, 2H), 9.48 (s, 1H).
(4). The compound (3) (0.77mmol), cyanoacetic acid (0.08g, 0.93mmol) and piperidine (0.92ml, 0.93mmol) were melted by acetonitril (20ml) and refluxed for four hours under nitrogen atmosphere. An organic layer was extracted from the mixture by using methylenechloride and water, evaporated and went through a column chromatography to obtain a compound 161. (eluent. EA : EtOH = 10:1) 1H NMR(DMSO) : [ppm] = 0.96 (m, 6H), 1.29 (m, 4H), 1.44 (s, 12H), 1.96 (m, 4H), 2.55 (m, 8H), 2.62 (m, 4H), 6.7 (m, 2H), 6.91 (m, 2H), 6.96 (m, 1H), 7.08 (s, 1H), 7.20 (m, 4H), 7.28 (m, 4H), 7.53 (m, 4H), 7.82 (d, ³J_{HH} = 8.8Hz, 2H), 11.51 (s, 1H).

### [Exemplary embodiment 18] Synthesis of compound 162

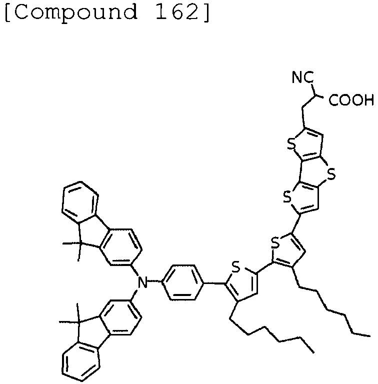

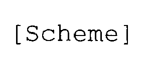

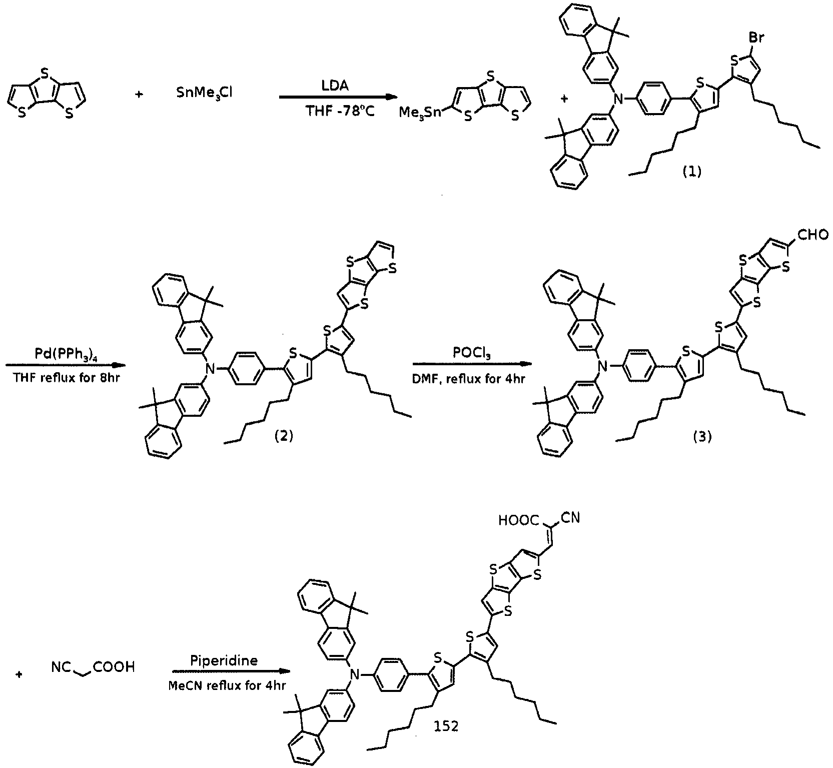

(1). Synthesized dithieno[2',3']thiophene (7g, 35.65mmol) was melted by THF (50ml), gradually added with LDA 2M (39ml) at -78°C, agitated for one hour at low temperatures, gradually added with trimethyltin chloride 1M (38ml) at -78°C, agitated for one hour at low temperatures and additionally agitated for 30 minutes at 0°C. After the agitation, an organic layer was extracted from the mixture by using methylenechloride and water, evaporated and dried. 1H NMR(CDCl₃) : [ppm] = 0.58 (s, 9H), 6.98 ((d, ³J_{HH} = 4.8Hz, 1H), 7.10 (s, 1H), 7.18 (d, ³J_{HH} = 4.8Hz, 1H).
(2). 2-trimethyl(dithieno[2',3']thiophene-2-yl)stannane (0.48g, 1.34mmol), the compound (1) (1g, 1.12mmol), and Pd(PPh₃)₄ (0.065g, 0.056mmol) were melted by THF (40ml) and refluxed for eight hours under nitrogen atmosphere. Then, an organic layer was extracted from the mixture by using methylenechloride and water, evaporated and went through a column chromatography. (eluent. M.C : Hx = 1:5) 1H NMR(CDCl₃) : [ppm] = 0.96 (m, 6H), 1.29 (m, 4H), 1.44 (s, 12H), 1.96 (m, 4H), 2.55 (m, 8H), 2.62 (m, 4H), 6.7 (m, 2H), 6.91 (m, 2H), 6.96 (d, ³J_{HH} = 4.8Hz, 1H), 7.08 (d, ³J_{HH} = 4.8Hz, 1H), 7.18 (m, 4H), 7.38 (m, 6H), 7.55 (m, 4H), 7.82 (d, ³J_{HH} = 8.8Hz, 2H).
(3). The compound (2) (1.24g, 1.2mmol) was melted by DMF (20ml), gradually added with phosphorous oxychloride (0.13ml, 1.44mmol) at 0°C and agitated for four hours at 80°C. After the agitation, an organic layer was extracted from the mixture by using methylenechloride and water, evaporated and went through column chromatography. (eluent. M.C : Hx = 1:4) 1H NMR (CDCl₃) : [ppm] = 0.96 (m, 6H), 1.29 (m, 4H), 1.44 (s, 12H), 1.96 (m, 4H), 2.55 (m, 8H), 2.62 (m, 4H), 6.7 (m, 1H), 6.91 (m, 2H), 7.08 (s, 1H), 7.18 (m, 6H), 7.38 (m, 6H), 7.54 (m, 4H), 7.78 (d, ³J_{HH} = 8.8Hz, 2H), 9.51 (s, 1H).
(4). The compound (3) (0.8g, 0.77mmol), cyanoacetic acid (0.08g, 0.93mmol) and piperidine (0.92ml, 0.93mmol) were melted by acetonitril (20ml) and refluxed for four hours under nitrogen atmosphere. An organic layer was then extracted from the mixture by using methylenechloride and water, evaporated and went through a column chromatography to obtain a compound 162. (eluent. EA : EtOH = 10:1) 1H NMR (DMSO) : [ppm] = 0.96 (m, 6H), 1.29 (m, 4H), 1.44 (s, 12H), 1.96 (m, 4H), 2.55 (m, 8H), 2.62 (m, 4H), 6.68 (s, 1H), 6.91 (m, 2H), 7.08 (m, 2H), 7.15 (m, 6H), 7.32 (m, 6H), 7.55 (m, 4H), 7.80 (d, ³J_{HH} = 8.8Hz, 2H), 11.51 (s, 1H).

### [Exemplary embodiment 19] Synthesis of compound 160

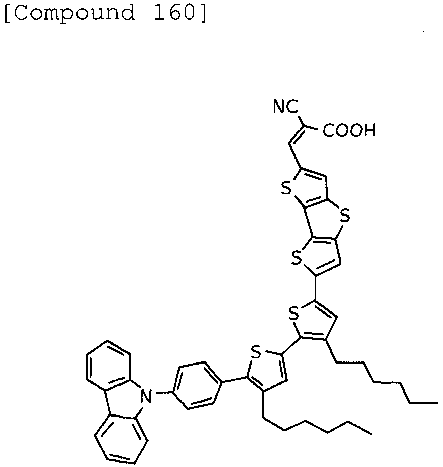

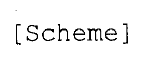

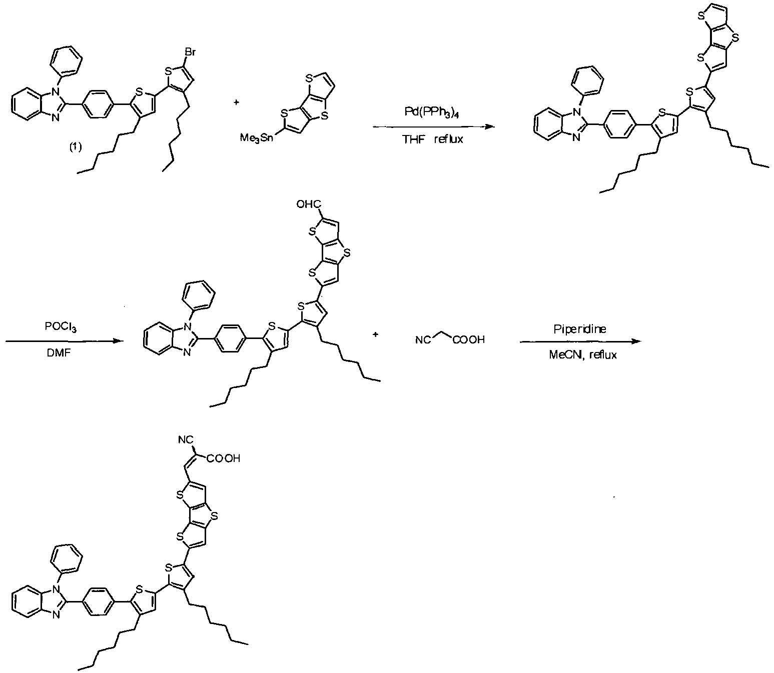

2-trimethyl(dithieno[2',3']thiophene-2-yl)stannane (0.48g, 1.34mmol), the compound (1) (0.76g, 1.12mmol), and Pd(PPh₃)₄ (0.065g, 0.056mmol) were melted by THF (40ml), and then synthesized and purified according to the synthesis method. Detailed synthesis and purification methods applied to those of the compound 161 according to the exemplary embodiment 17.

### [Exemplary embodiment 20] Synthesis of compound 175

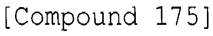

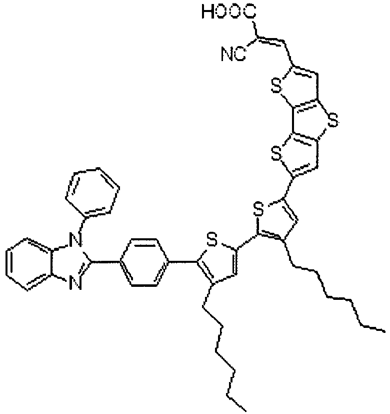

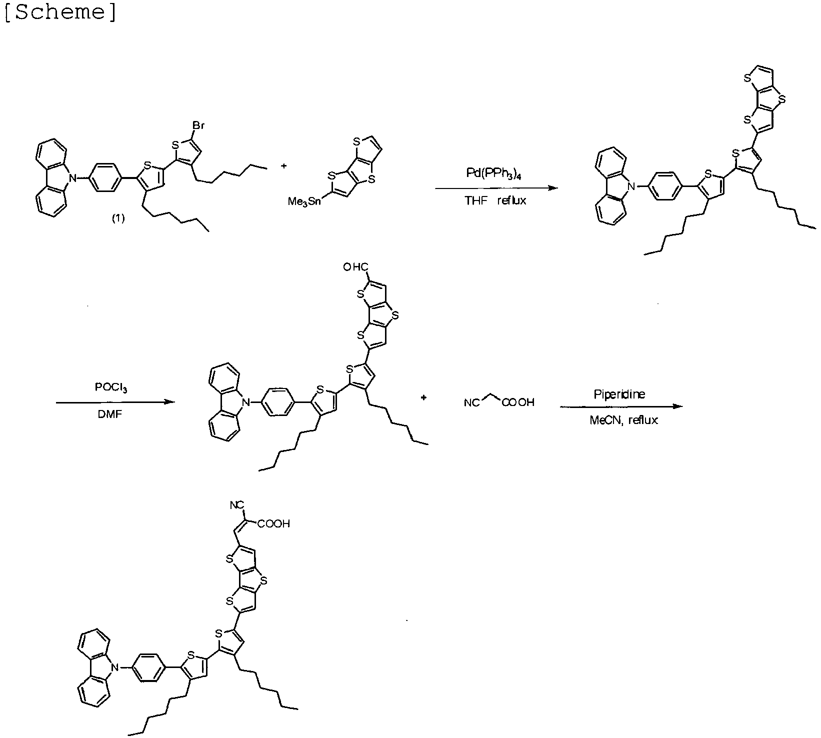

2-trimethyl(dithieno[2',3']thiophene-2-yl)stannane (0.48g, 1.34mmol), the compound (1) (0.73g, 1.12mmol), and Pd(PPh₃)₄ (0.065g, 0.056mmol) were melted by THF (40ml), and then synthesized and purified according to the synthesis method. Detailed synthesis and purification methods applied to those of the compound 161 according to the exemplary embodiment 17.

### [Exemplary embodiment 21] Fabrication of dye-sensitized solar cell

A solar cell was manufactured by using a 12+8*µ*m TiO₂ transparent layer to evaluate current-voltage properties of the dye compounds according to the present invention. A TiO₂ paste (solaronix, 13nm paste) was screen-printed to make a first TiO₂ layer in 12*µ*m, and a second TiO₂ dispersion layer having a thickness of 8*µ*m was made by using another paste (CCIC, HWP-400) for light dispersion. The TiO₂ double layer was treated by 40mN TiCl₄ liquid and dried for 30 minutes at 500°C. After cooling the treated film into 60°C, the film was applied with each solution of the dye compounds 1, 6, 8, 28, 95, 127, 142, 147, 148, 149, 150, 161 and 162 (0.3mM dye of 10mM kenodioxycholic acid containing ethanol). The dye-absorbed TiO₂ electrode was coupled with a sandwich cell which was heated and sealed, leaving a high temperature molten film (Surlyn 1702, 25*µ*m) as a spacer between platinum and counter electrode. The electrolyte liquid used was 0.6M 3-hexyl-1,2-dimethylimidazolium iodine, 0.04M I₂, 0.025M LiI, 0.05M guanidium thiocyanate and 0.28M tert-butylpiridine of acetonitrile.

### [Exemplary embodiment 22] Measurement of properties of dye and dye-sensitized solar cell prepared

The properties of the solar cell which was manufactured according to the exemplary embodiment 21 were measured and the result was shown in Table 1.

**[Table 1]**

| | Voc (V) | Isc(A) | Jsc (mA/ cm²) | Fill factor (%) | Test element dimension (cm²) | Effici ency (%) |
|---|---|---|---|---|---|---|
| Compound 1 | 0.603 | 0.0010669 | 4.559 | 73.60 | 0.234 | 2.02 |
| Compound 2 | 0.595 | 0.0016780 | 6.726 | 70.56 | 0.249 | 2.83 |
| Compound 3 | 0.518 | 0.0035876 | 1.429 | 73.84 | 0.251 | 0.55 |
| Compound 4 | 0.523 | 0.0001490 | 0.558 | 57.75 | 0.267 | 0.17 |
| Compound 5 | 0.560 | 0.0029756 | 1.240 | 72.27 | 0.240 | 0.58 |
| Compound 6 | 0.564 | 0.0019752 | 8.162 | 70.33 | 0.242 | 3.23 |
| Compound 8 | 0.732 | 0.0020896 | 8.358 | 67.62 | 0.250 | 4.14 |
| Compound 28 | 0.668 | 0.00213 | 8.148 | 70.62 | 0.262 | 3.84 |
| Compound 95 | 0.682 | 0.00193 | 7.47 | 69.60 | 0.258 | 3.54 |
| Compound 127 | 0.54 | 0.00254 | 9.61 | 71.5 | 0.264 | 3.71 |
| Compound 142 | 0.569 | 0.00150 | 6.065 | 72.36 | 0.248 | 2.50 |
| Compound 147 | 0.759 | 0.00260 | 10.08 | 59.6 | 0.258 | 4.46 |
| Compound 148 | 0.654 | 0.00162 | 14.96 | 76.4 | 0.116 | 6.97 |
| Compound 149 | 0.631 | 0.00144 | 10.19 | 75.0 | 0.141 | 4.82 |
| Compound 150 | 0.579 3 | 0.00264 | 9.982 | 70.86 | 0.264 | 4.10 |
| Compound 161 | 0.653 | 0.00177 | 12.07 | 71.3 | 0.147 | 5.62 |
| Compound 162 | 0.699 | 0.00268 | 13.45 | 74 | 0.199 | 8.28 |
| Ref. (N-719) | 0.775 | 0.00390 | 18.04 | 68 | 0.216 | 9.51 |

As seen from the result above, the dye according to the present invention provides good molar extinction coefficient, J_{sc}(short-circuit photocurrent density) and photoelectric conversion efficiency to drastically enhance efficiency of a solar cell.

Although a few exemplary embodiments of the present invention have been shown and described, it will be appreciated by those skilled in the art that changes may be made in these exemplary embodiments without departing from the scope, which is defined in the appended claims.

### [Industrial Applicability]

A thiophene-based dye according to the present invention may greatly enhance efficiency of a solar cell by providing better molar extinction coefficient, J_{sc}(short-circuit photocurrent density) and photoelectric conversion efficiency than a conventional metal complex dye and drastically lower dye synthesis cost by being purified without expensive column.

## Claims

1. A dithienothiophene-based dye which comprises one of the compounds represented by compounds 158 to 180 below:

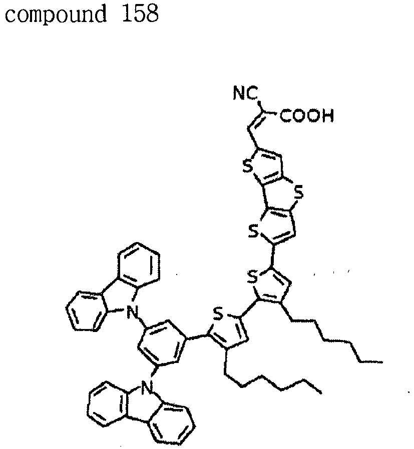

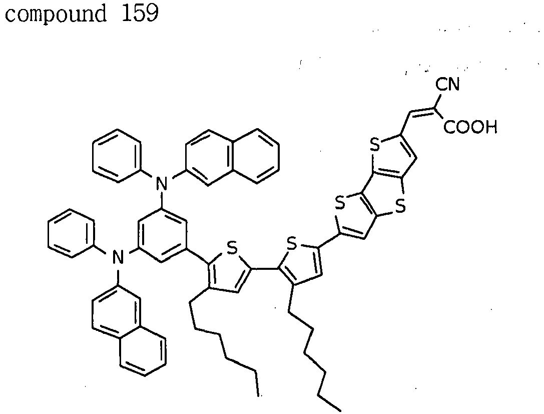

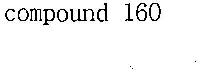

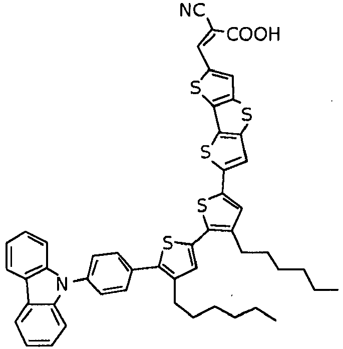

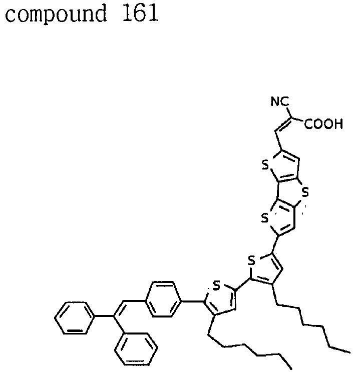

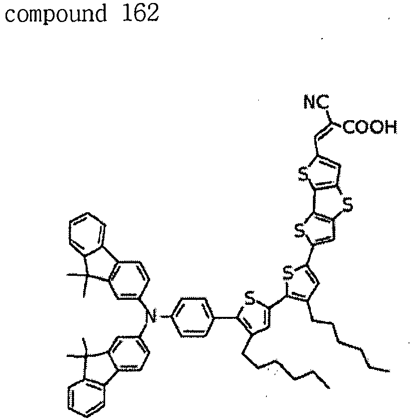

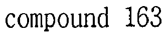

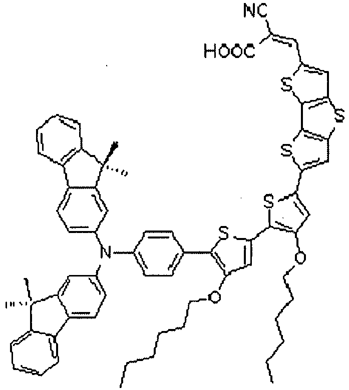

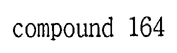

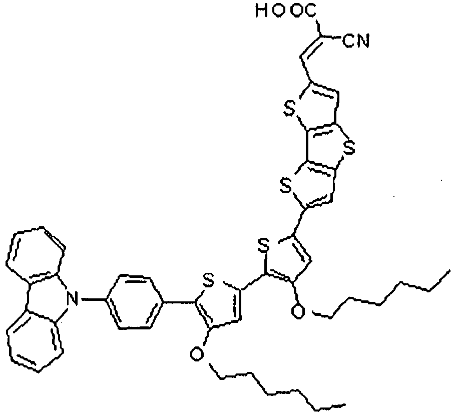

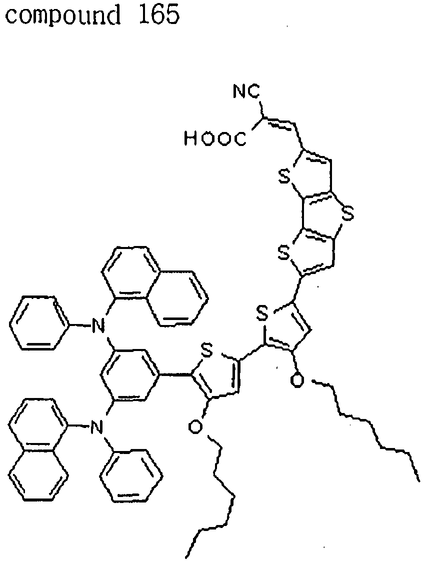

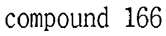

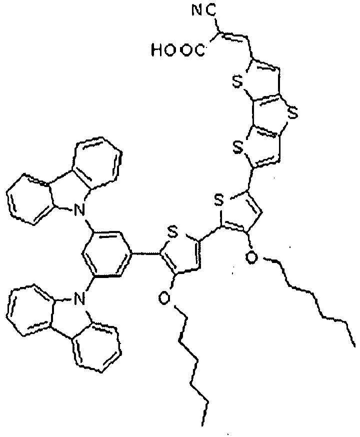

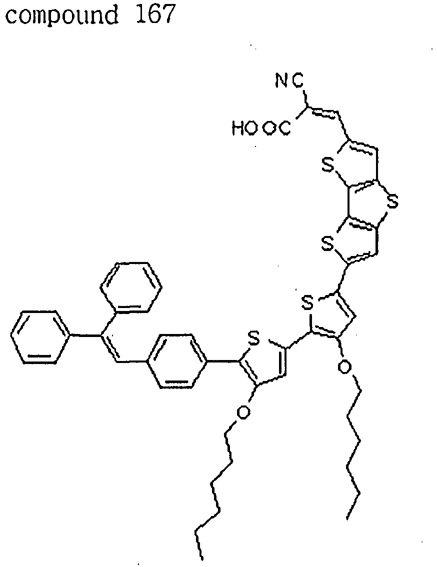

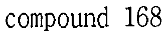

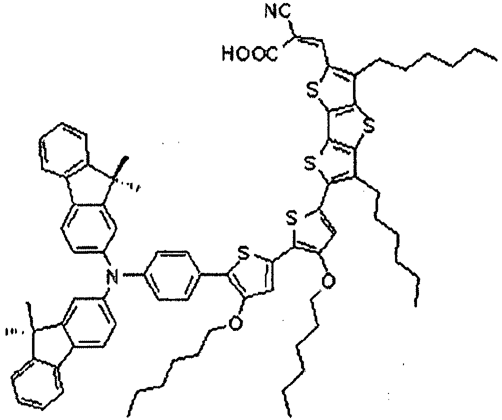

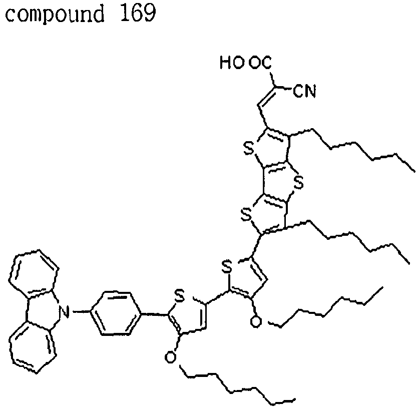

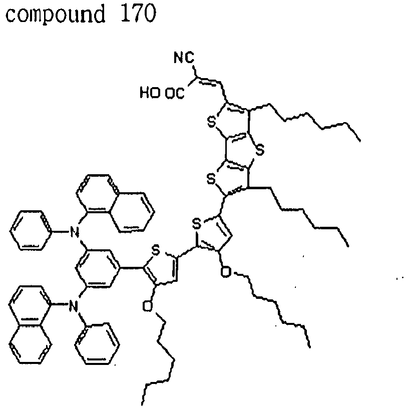

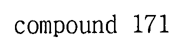

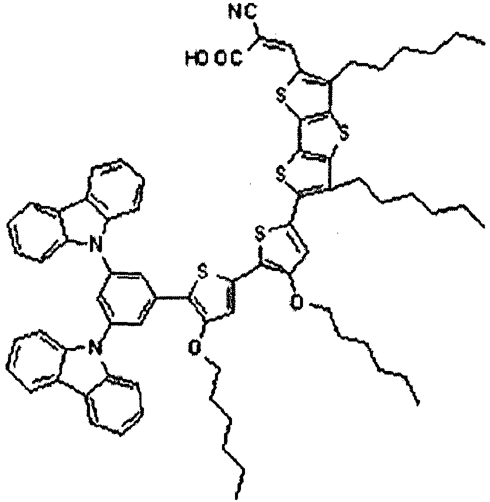

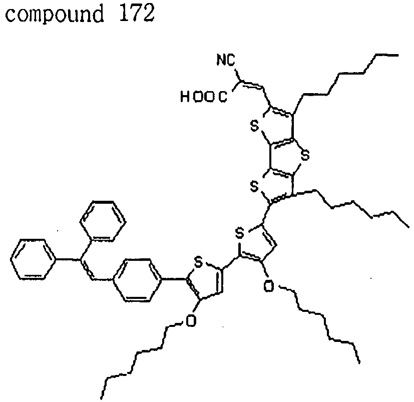

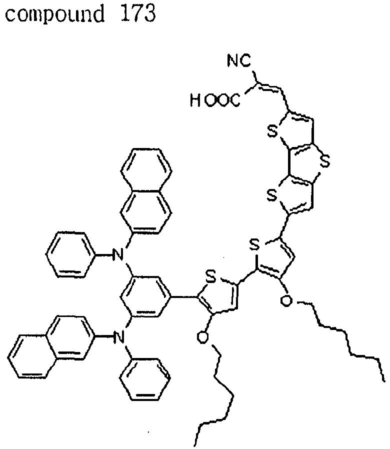

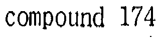

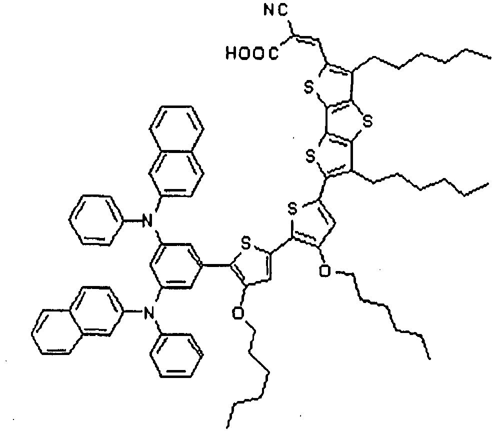

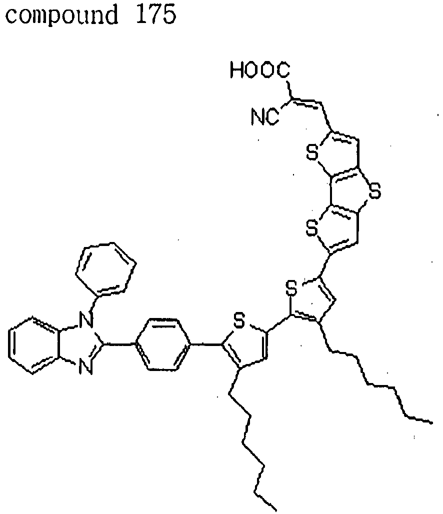

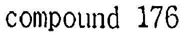

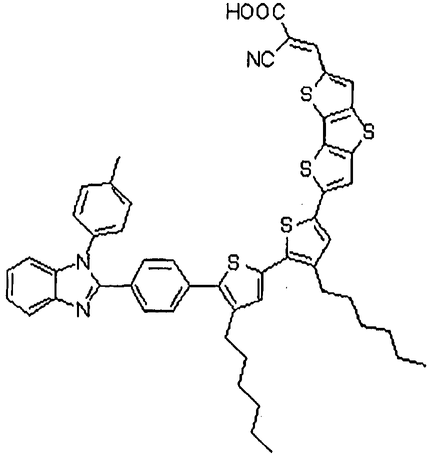

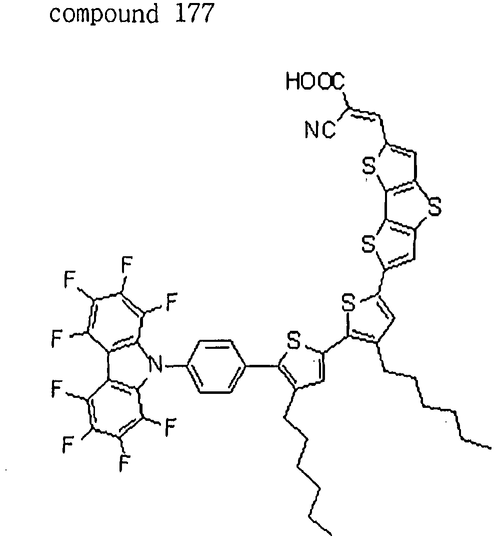

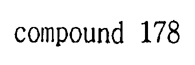

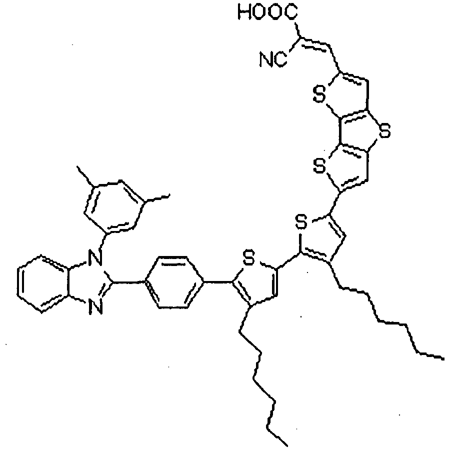

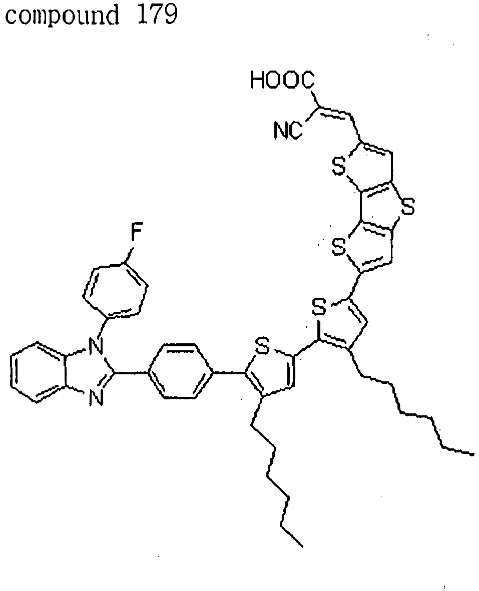

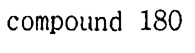

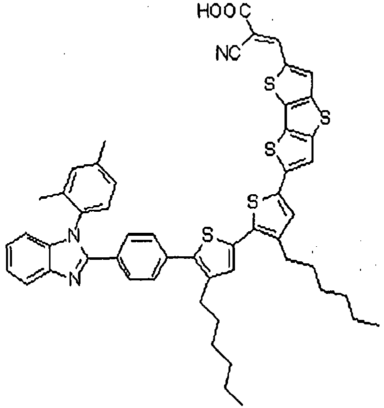

2. A dye-sensitized photoelectric conversion element which comprises oxide semiconductor particles applied with the dye according to claim 1.

3. The dye-sensitized photoelectric conversion element according to claim 2, wherein the oxide semiconductor particles are applied with a thiophene-based dye under a presence of an inclusion compound.

4. The dye-sensitized photoelectric conversion element according to claim 2, wherein the oxide semiconductor particles comprise titanium dioxide as a compulsory component.

5. The dye-sensitized photoelectric conversion element according to claim 2, wherein the oxide semiconductor particles have an average diameter of 1 to 500nm.

6. A dye-sensitized solar cell which comprises the dye-sensitized photoelectric conversion element according to claim 2 as an electrode.

7. The dye-sensitized solar cell according to claim 6, which is prepared by an operation of coating TiO₂ paste on a transparent conductive substrate, an operation of firing the paste-coated substrate to form a TiO₂ layer, an operation of dipping the substrate having the TiO₂ layer into a solution including the dissolved dye to form a TiO₂ film electrode having the dye, an operation of providing a second glass substrate including a counter electrode on the TiO₂ film electrode, an operation of forming a hole to pass through the second glass substrate and the counter electrode, an operation of coupling the counter electrode and the TiO₂ film electrode by heat and pressure, leaving a thermoplastic polymer film therebetween, an operation of injecting an electrolyte to the thermoplastic polymer film interposed between the counter electrode and the TiO₂ film electrode through the hole and an operation of sealing the thermoplastic polymer film.

## Patentansprüche

1. Dithienothiophen-basierter Farbstoff, der eine der Verbindungen aufweist, die durch nachstehende Verbindungen 158 bis 180 dargestellt sind:

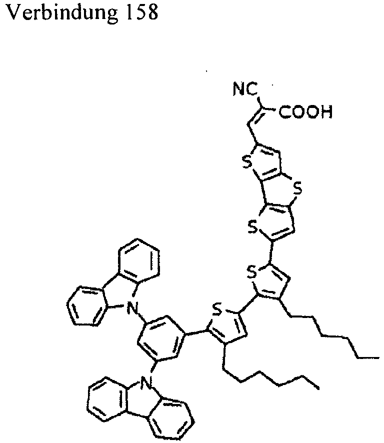

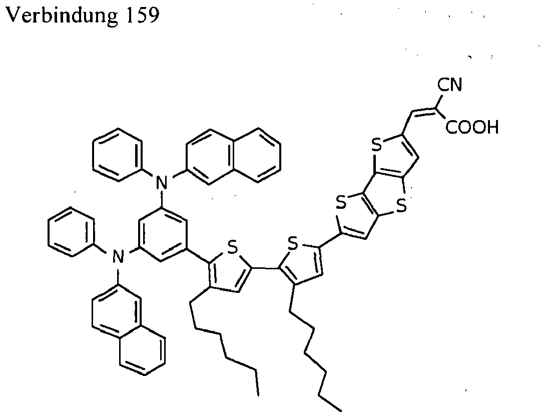

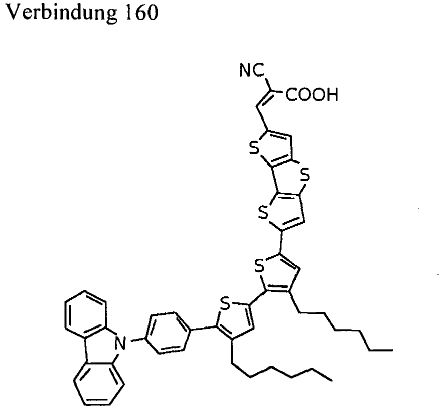

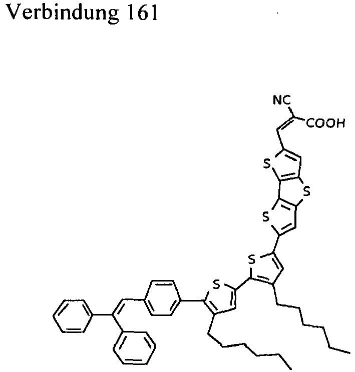

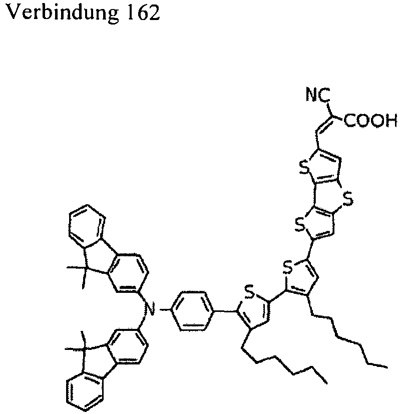

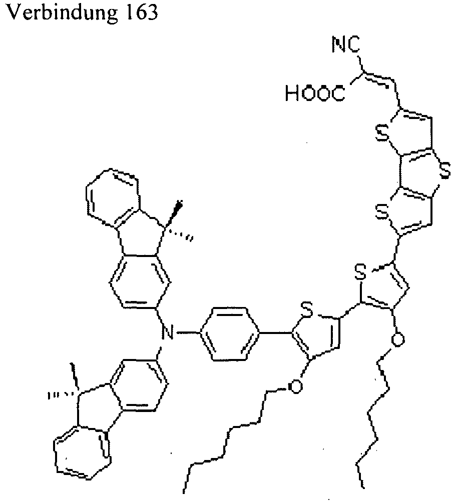

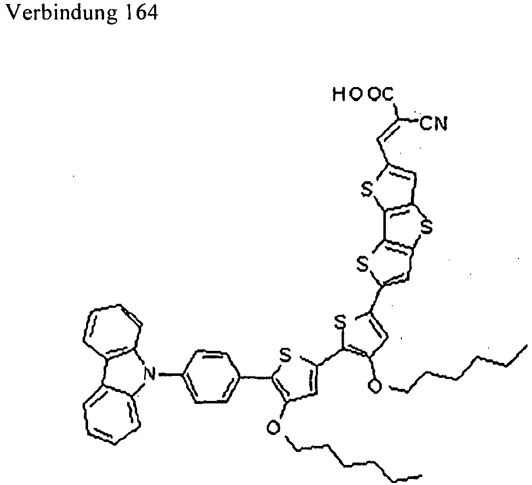

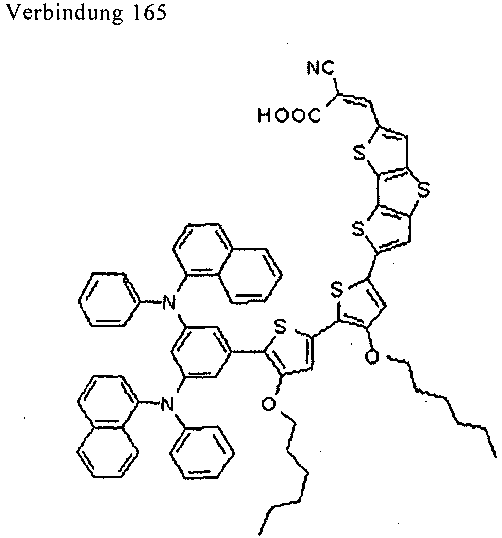

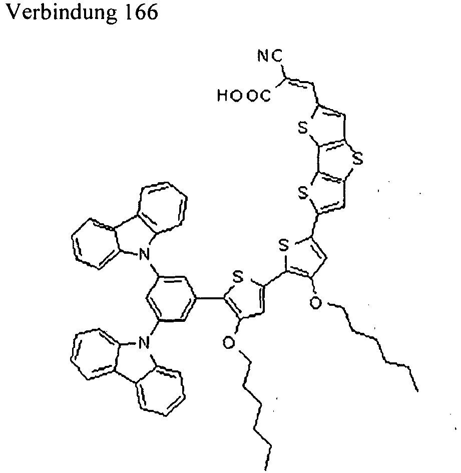

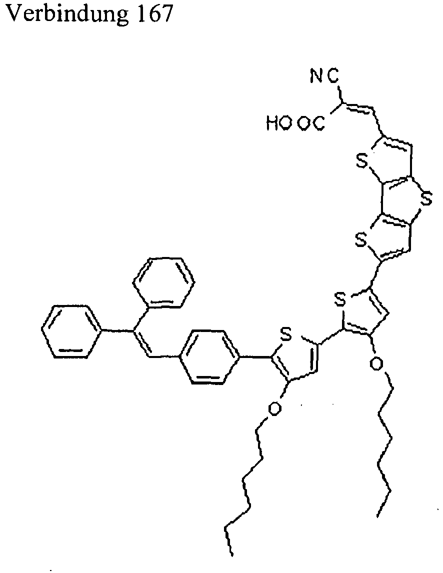

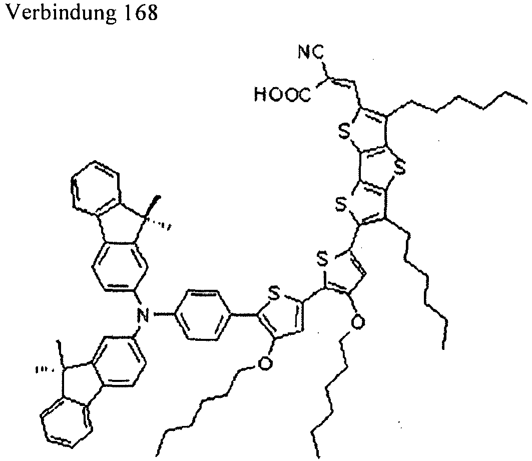

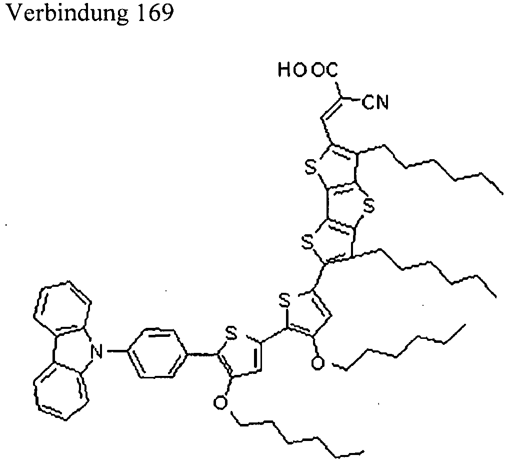

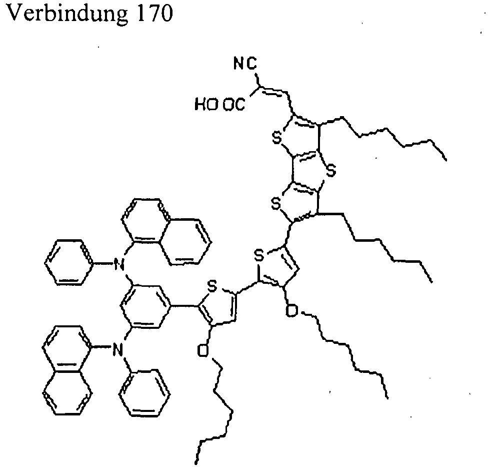

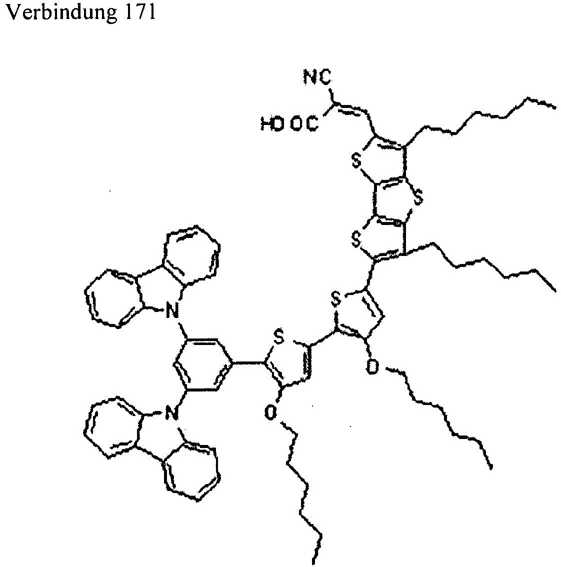

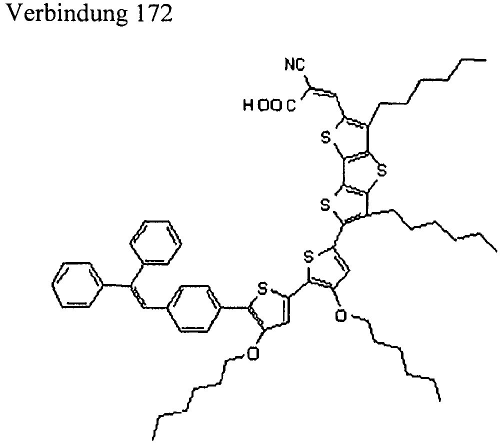

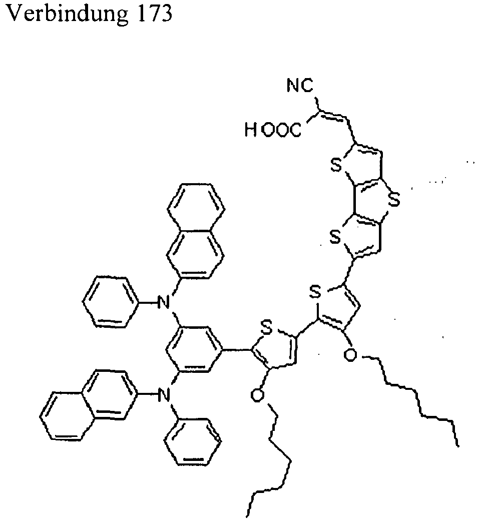

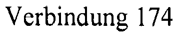

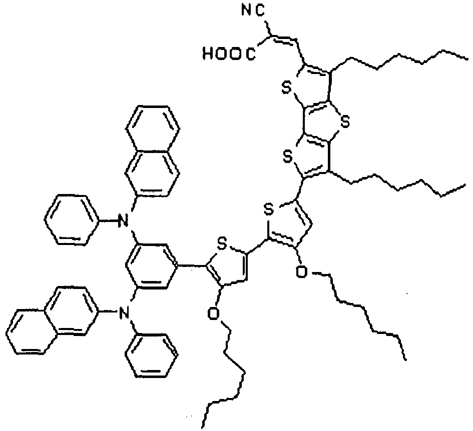

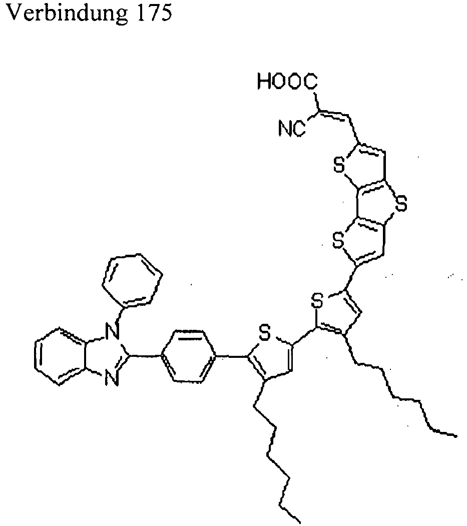

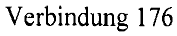

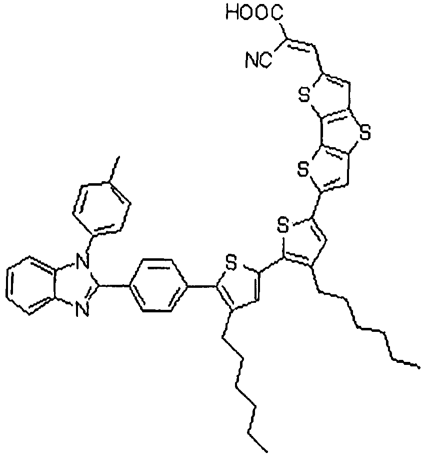

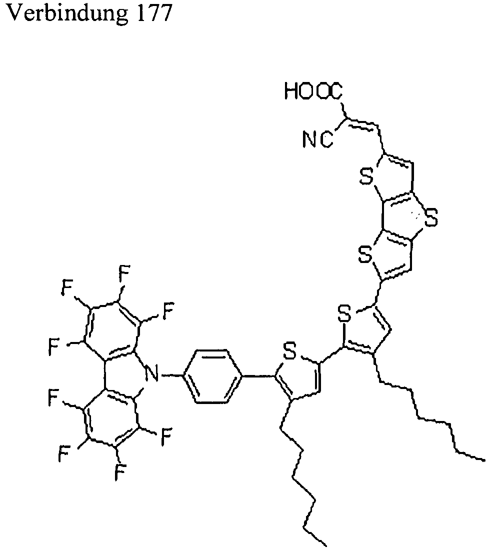

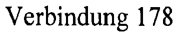

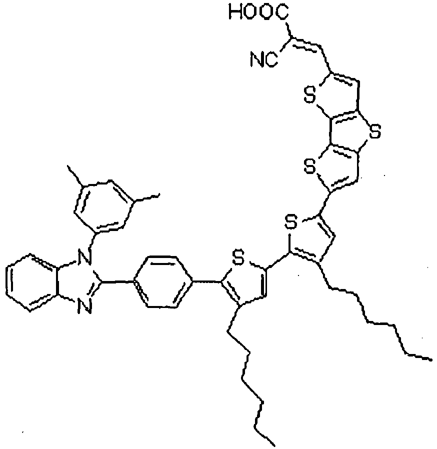

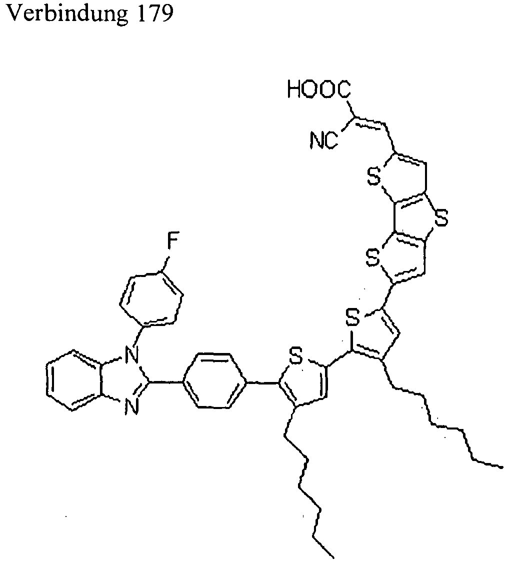

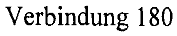

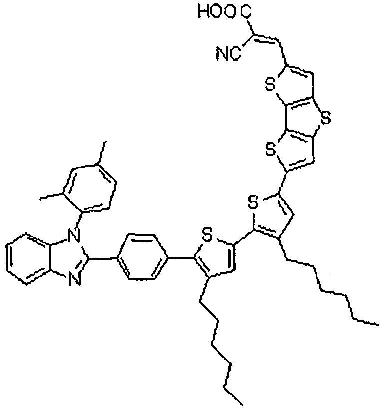

2. Farbstoffsensibilisiertes photoelektrisches Umwandlungselement, das Oxid-Halbleiter-Partikel aufweist, die mit dem Farbstoff gemäß Anspruch 1 bereitgestellt sind.

3. Farbstoffsensibilisiertes photoelektrisches Umwandlungselement gemäß Anspruch 2, wobei die Oxid-Halbleiter-Partikel mit einem Farbstoff auf Thiophenbasis in Gegenwart einer Einschlussverbindung bereitgestellt sind.

4. Farbstoffsensibilisiertes photoelektrisches Umwandlungselement gemäß Anspruch 2, wobei die Oxid-Halbleiter-Partikel Titandioxid als eine obligatorische Komponente aufweisen.

5. Farbstoffsensibilisiertes photoelektrisches Umwandlungselement gemäß Anspruch 2, wobei die Oxid-Halbleiter-Partikel einen durchschnittlichen Durchmesser von 1 bis 500nm aufweisen.

6. Farbstoffsensibilisierte Solarzelle, die das farbstoffsensibilisierte photoelektrische Umwandlungselement gemäß Anspruch 2 als eine Elektrode aufweist.

7. Farbstoffsensibilisierte Solarzelle gemäß Anspruch 6, die hergestellt ist durch einen Vorgang des Auftragens einer TiO₂-Paste auf ein transparentes leitfähiges Substrat, einen Vorgang des Feuerns des Pasten-beschichteten Substrats zur Bildung einer TiO₂-Schicht, einen Vorgang des Tauchens des Substrats mit der TiO₂-Schicht in eine Lösung, die den gelösten Farbstoff enthält, um eine TiO₂-Filmelektrode mit dem Farbstoff zu bilden, einen Vorgang des Bereitstellens eines zweiten Glassubstrats einschließlich einer Gegenelektrode auf der TiO₂-Filmelektrode, einen Vorgang des Bildens eines Lochs zum Verlaufen durch das zweite Glassubstrat und die Gegenelektrode, einen Vorgang des Verbindens der Gegenelektrode und der TiO₂-Filmelektrode durch Wärme und Druck, hinterlassend einen thermoplastischen Polymerfilm dazwischen, einen Vorgang des Spritzens eines Elektrolyts auf den thermoplastischen Polymerfilm, der zwischen der Gegenelektrode und TiO₂-Filmelektrode liegt, durch das Loch und einen Vorgang des Abdichtens des thermoplastischen Polymerfilms.

## Revendications

1. Colorant à base de dithiénothiophène qui comprend l'un des composés représentés par les composés 158 à 180 ci-dessous :

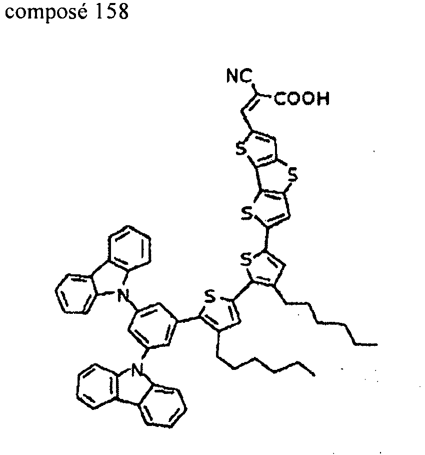

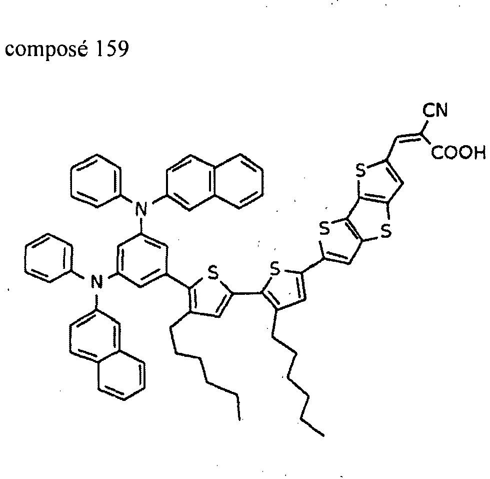

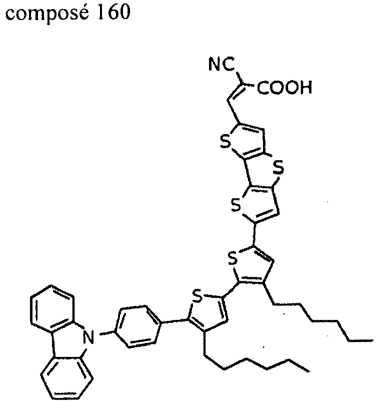

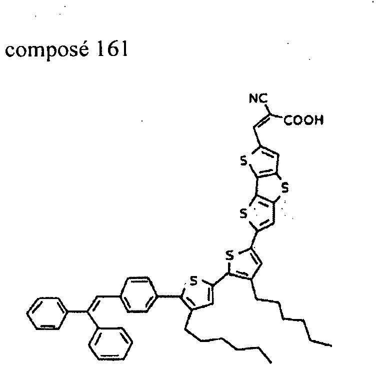

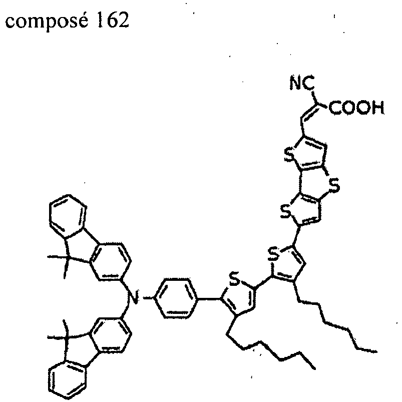

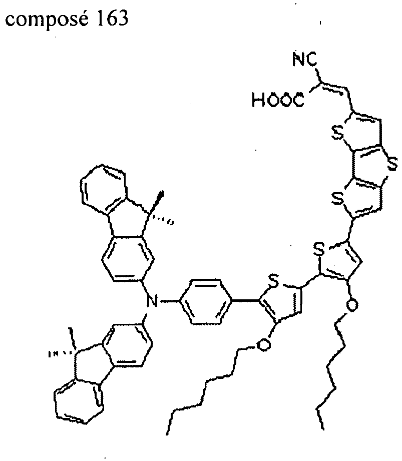

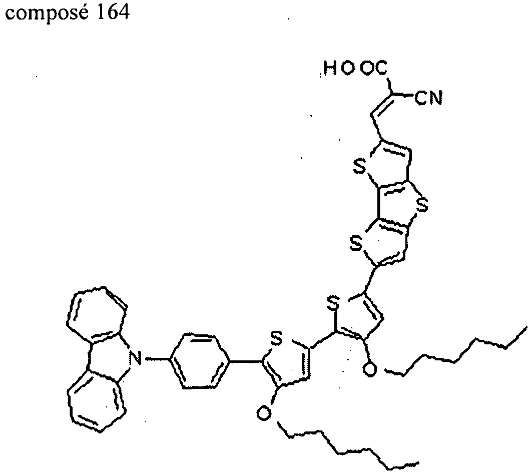

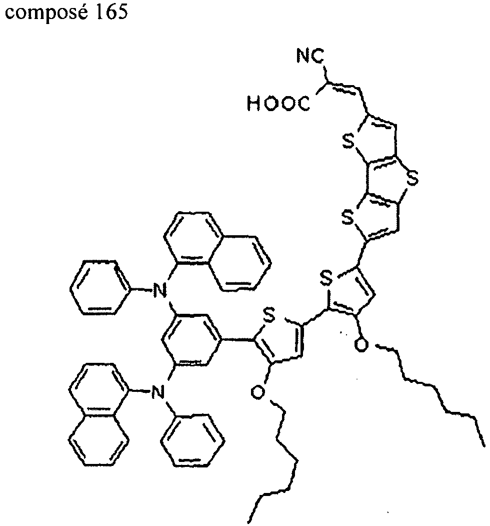

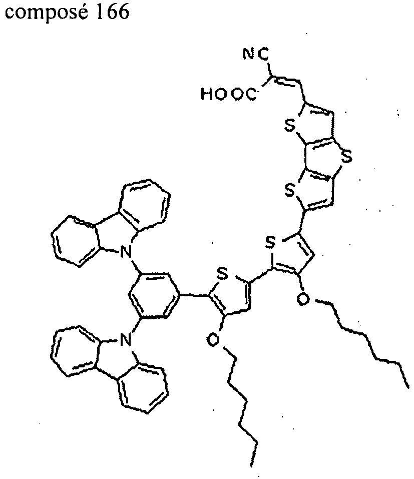

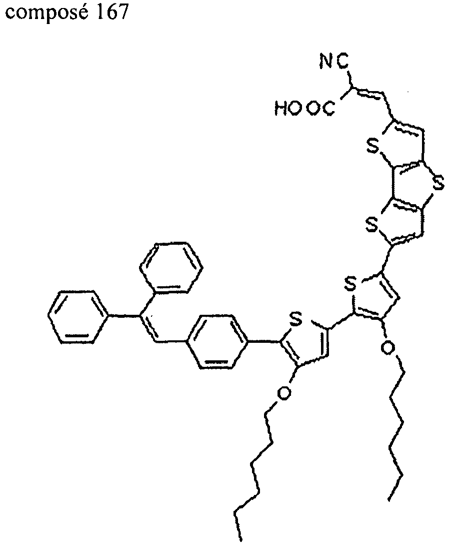

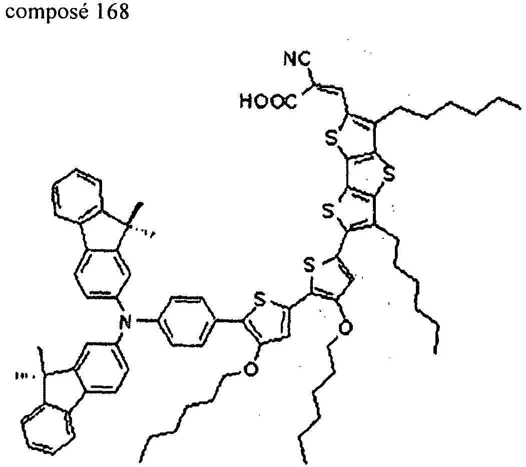

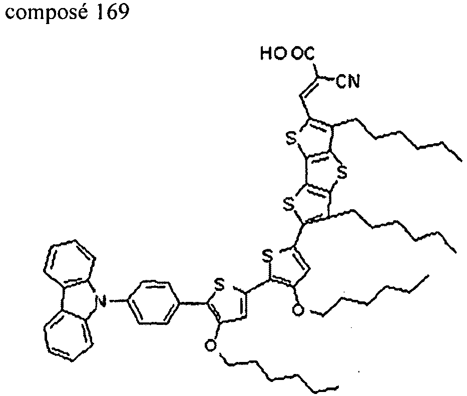

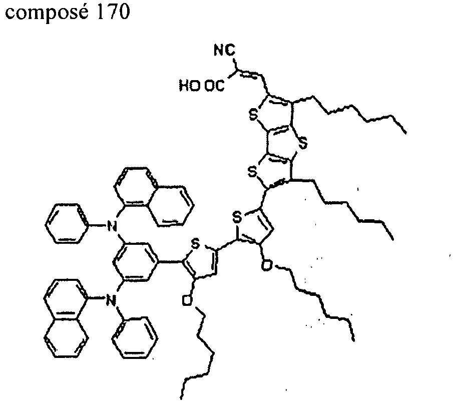

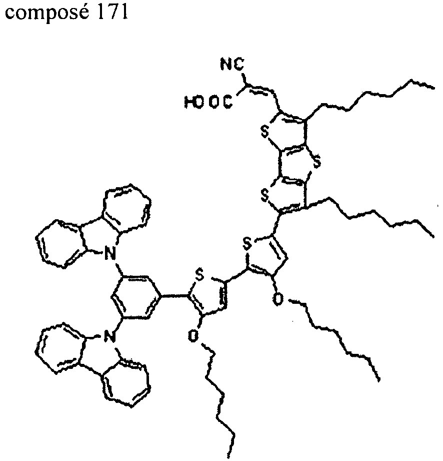

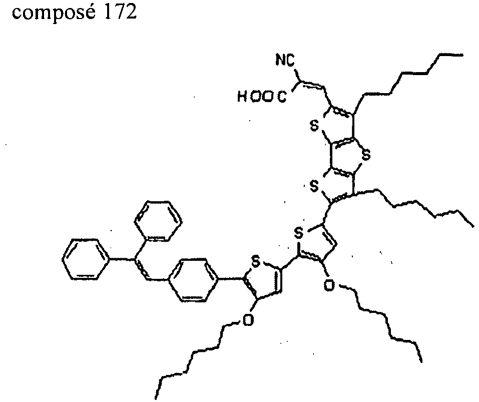

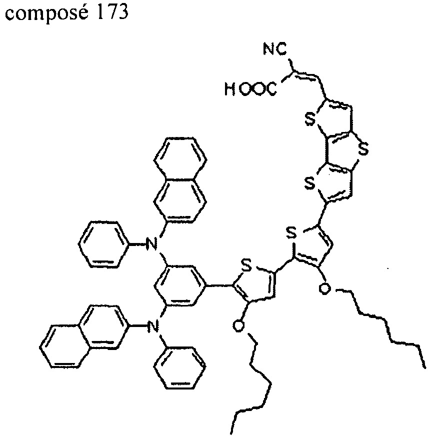

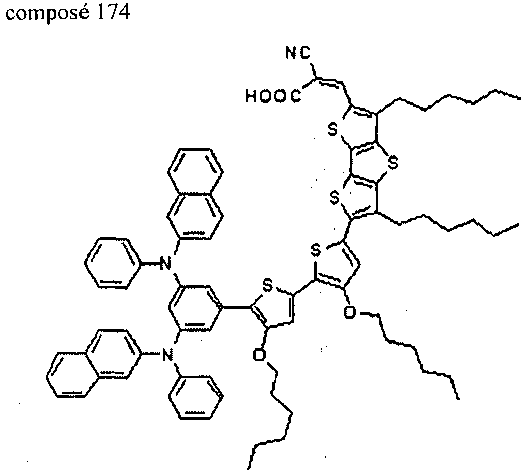

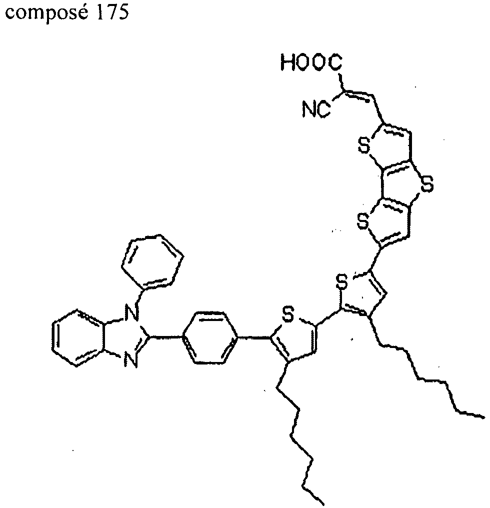

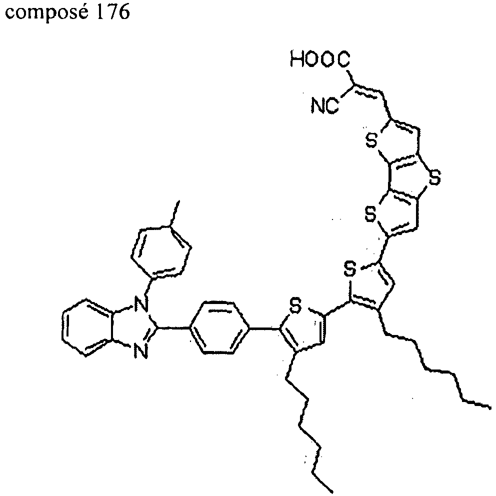

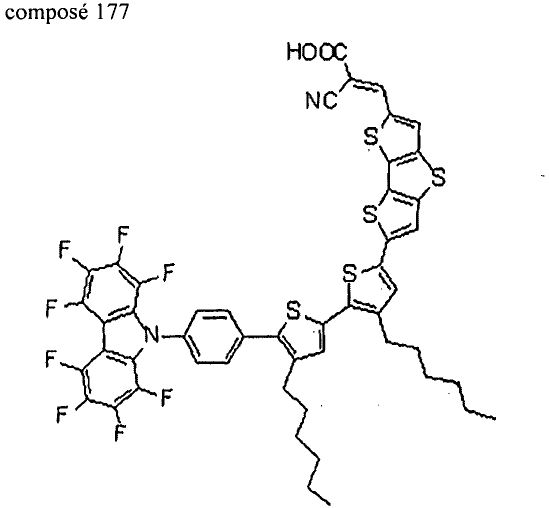

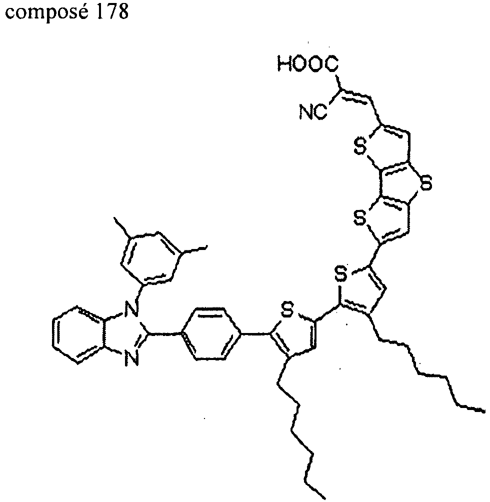

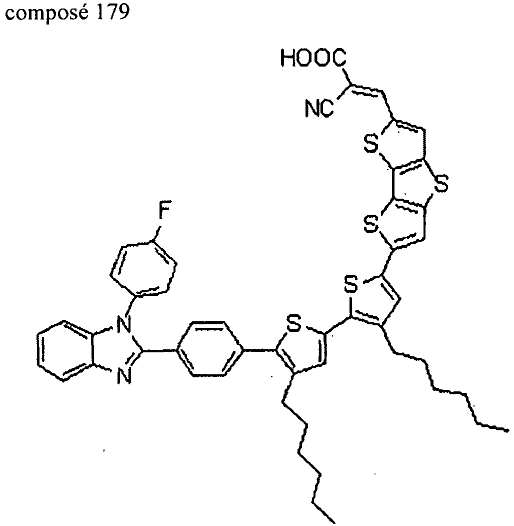

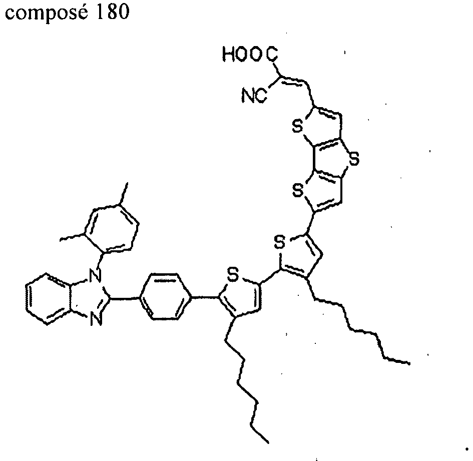

2. Elément de conversion photoélectrique sensibilisé par un colorant qui comprend des particules d'oxyde semi-conducteur auquel le colorant selon la revendication 1 est appliqué.

3. Elément de conversion photoélectrique sensibilisé par un colorant selon la revendication 2, dans lequel les particules d'oxyde semi-conducteur sont appliquées avec un colorant à base de thiophène en présence d'un composé d'inclusion.

4. Elément de conversion photoélectrique sensibilisé par un colorant selon la revendication 2, dans lequel les particules d'oxyde semi-conducteur comprennent du dioxyde de titane en tant que composant obligatoire.

5. Elément de conversion photoélectrique sensibilisé par un colorant selon la revendication 2, dans lequel les particules d'oxyde semi-conducteur ont un diamètre moyen de 1 à 500 nm.

6. Cellule solaire sensibilisée par un colorant qui comprend l'élément de conversion photoélectrique sensibilisé par le colorant selon la revendication 2 en tant qu'électrode.

7. Cellule solaire sensibilisée par un colorant selon la revendication 6, qui est préparée par une opération de revêtement d'une pâte de TiO₂ sur un substrat conducteur transparent, une opération de cuisson du substrat revêtu de pâte pour former une couche de TiO₂, une opération d'immersion du substrat comportant la couche de TiO₂ dans une solution incluant le colorant dissout pour former une électrode à film de TiO₂ comportant le colorant, une opération de fourniture d'un second substrat de verre incluant une contre-électrode sur l'électrode à film de TiO₂, une opération de formation d'un trou pour traverser le second substrat de verre et la contre-électrode, une opération de couplage de la contre-électrode et de l'électrode à film de TiO₂ par la chaleur et la pression, laissant entre elles un film polymère thermoplastique, une opération d'injection d'un électrolyte dans le film polymère thermoplastique interposé entre la contre-électrode et l'électrode à film de TiO₂ à travers le trou et une opération de scellage du film polymère thermoplastique.
